(19) Europäisches Patentamt
European Patent Office
Office européen des brevets

(11) EP 2 028 712 A2

(12) EUROPEAN PATENT APPLICATION

(43) Date of publication:
25.02.2009 Bulletin 2009/09

(51) Int Cl.:
H01M 8/16 (2006.01)

(21) Application number: 08162292.0

(22) Date of filing: 13.08.2008

(84) Designated Contracting States:
AT BE BG CH CY CZ DE DK EE ES FI FR GB GR
HR HU IE IS IT LI LT LU LV MC MT NL NO PL PT
RO SE SI SK TR
Designated Extension States:
AL BA MK RS

(30) Priority: 16.08.2007 US 956305 P
01.08.2008 US 184820

(71) Applicant: Sony Corporation
Tokyo 108-0075 (JP)

(72) Inventors:
• Sakai, Hideki
  TOKYO 108-0075 (JP)
• Nakagawa, Takaaki
  TOKYO 108-0075 (JP)
• Tokita, Yuichi
  TOKYO 108-0075 (JP)
• Hatazawa, Tsuyonobu
  TOKYO 108-0075 (JP)

(74) Representative: Beder, Jens
Mitscherlich & Partner
Patent-und Rechtsanwälte
Sonnenstraße 33
80331 München (DE)

(54) Biofuel cell, method for producing the same, electronic apparatus, enzyme-immobilized electrode, and method for producing the same

(57) A biofuel cell has a structure in which a cathode and an anode are opposed to each other with a electrolyte layer provided therebetween, at least one of the cathode and the anode including an electrode on which at least one enzyme and at least one electron mediator are immobilized. The concentration of the electron mediator immobilized on the electrode is at least 10 times a Michaelis constant $K_m$ of the electron mediator for the enzyme, which is determined by measurement in a solution.

**Description**

BACKGROUND

The present invention relates to a biofuel cell, a method for producing the same, an electronic apparatus using the biofuel cell as a power supply, an enzyme-immobilized electrode, and a method for producing the same.

Biofuel cells can generate electric power under mild conditions (room temperature and neutral pH) using a renewable energy source such as sugar, an organic acid, or the like, i.e., a combination of oxidation of fuel and reduction of oxygen ($O_2$), in the presence of an enzyme or microorganisms as a catalyst (refer to, for example, Non-Patent Documents 1 to 14). The biofuel cells are expected as next-generation safe energy devices with higher energy density as compared with current cells such as lithium ion cells and direct methanol fuel cells. The power densities of small biofuel cells proposed as implantable power supplies for medical devices are on the order of sub-mW/$cm^2$ even using mechanical stirring or a convective system for fuel and oxygen, such as a pump, and the power per single biofuel cell is only several $\mu$W (refer to Non-Patent Documents 1 and 10). When a convective system is not used, the power density is decreased by several orders due to the limitation of material transport rates of fuel and oxygen and the material permeability barrier in a catalyst layer fixed on an electrode (refer to Non-Patent Document 15).

The electric power necessary for mobile electronic apparatuses is at least 100 mW depending on the apparatuses and operating conditions, and this power is sufficient for, for example, operating portable music players and the like. In order to achieve such power, the inventors of the present invention have proposed an electron mediator-type biofuel cell including an electron mediator for transferring electrons between an electrode and an enzyme (refer to, for example, Patent Documents 1 to 12).

[Non-Patent Document 1] Heller, A. Miniature biofuel cells. Phys. Chem. Chem. Phys. 6, 209-216 (2004)

[Non-Patent Document 2] Barton, S. C., Gallaway, J. & Atanassov, P. Enzymatic biofuel cells for implantable and microscale devices. Chem. Rev. 104, 4867-4886 (2004)

[Non-Patent Document 3] Palmore, G. T. R. & Whitesides, G. M. Microbial and enzymatic biofuel cells. ACS Symp. Ser. 556, 271-290 (1994)

[Non-Patent Document 4] Kano, K., Ikeda, T. Bioelectrocatalysis, Powerful means of connecting electrochemistry to biochemistry and biotechnology. Electrochemistry 71(2), 86-99 (2003)

[Non-Patent Document 5] Katz, E., & Willner, I. A biofuel cell with electrochemically switchable and tunable power output. J. Am. Chem. Soc. 125, 6803-6813 (2003)

[Non-Patent Document 6] Kamitaka, Y., Tsujimura, S., Setoyama, N., Kajino, T., & Kano, K. Fructose/dioxygen biofuel cell based on direct electron transfer-type bioelectrocatalysis. Phys. Chem. Chem. Phys. 9(15), 1793-1801 (2007)

[Non-Patent Document 7] Palmore, G. T. R., Bertschy, H., Bergens, S. H. & Whitesides, G. M. A methanol/dioxygen biofuel cell that uses NAD(+)-dependent dehydrogenases as catalysts: Application of an electro-enzymatic method to regenerate nicotinamide adenine dinucleotide at low overpotentials, J. Electroanal. Chem. 443, 155-161 (1998)

[Non-Patent Document 8] Topcagic, S., & Minteer, S. D. Development of a membraneless ethanol/oxygen biofuel cell. Electrochimica Acta 51, 2168-2172 (2007)

[Non-Patent Document 9] Tsujimura, S., Fujita, M., Tatsumi, H., Kano, K. & Ikeda, T. Bioelectrocatalysis-based dihydrogen/dioxygen fuel cell operating at physiological pH. Phys. Chem. Chem. Phys. 3, 1331-1335 (2001)

[Non-Patent Document 10] Mano, N., Mao, F. & Heller, A. Characteristics of a miniature compartment-less grucose-O2 biofuel cell and its operation in a living plant. J. Am. Chem. Soc. 125, 6588-6594 (2003)

[Non-Patent Document 11] Togo, M., Takamura, A., Asai, T., Kaji, H. & Nishizawa, M. An enzyme-based microfluidic biofuel cell using vitamin K3-mediated glucose oxidation, Electrochimica Acta 52, 4669-4674 (2007)

[Non-Patent Document 12] Chaudhuri, S. K. & Lovley, D. R. Electricity generation by direct oxidation of glucose in mediatorless microbial fuel cells, Nature Biotech. 21(10), 1229-1232 (2003)

[Non-Patent Document 13] Logan, B. E., Aelterman, P., Hamelers, B., Rozendal, R., Schroeder, U., Keller, J., Freguia, S., Verstraete, W. & Rabaey, K. Microbial fuel cells: Methodology and technology, Environmental Science & Technology 9(3), 5181-5192 (2006)

[Non-Patent Document 14] Bullen, R. A., Arnot, T. C., Lakeman, J. B. & Walsh, F. C. Biofuel cells and their development. Biosens. Bioelectron. 21, 2015-2045 (2006)

[Non-Patent Document 15] Sato, A., Kano, K. & Ikeda, T. Diaphorase/naphtoquinone derivative-modified electrode as an anode for diffusion-controlled oxidation of NADH in electrochemical cells. Chem. Lett. 32, 880-881 (2003)

[Patent Document 1] Japanese Unexamined Patent Application Publication No. 2004-71559

[Patent Document 2] Japanese Unexamined Patent Application Publication No. 2005-13210

[Patent Document 3] Japanese Unexamined Patent Application Publication No. 2005-310613

[Patent Document 4] Japanese Unexamined Patent Application Publication No. 2006-24555
[Patent Document 5] Japanese Unexamined Patent Application Publication No. 2006-49215
[Patent Document 6] Japanese Unexamined Patent Application Publication No. 2006-93090
[Patent Document 7] Japanese Unexamined Patent Application Publication No. 2006-127957
[Patent Document 8] Japanese Unexamined Patent Application Publication No. 2006-156354
[Patent Document 9] Japanese Unexamined Patent Application Publication No. 2007-12281
[Patent Document 10] Japanese Unexamined Patent Application Publication No. 2007-35437
[Patent Document 11] Japanese Unexamined Patent Application Publication No. 2007-87627
[Patent Document 12] Japanese Unexamined Patent Application Publication No. 2007-188810

However, the above-described electron mediator-type biofuel cell has been desired to be further improved in current density or power density.

Accordingly, a problem to be resolved by the present invention is to provide a biofuel cell capable of being significantly improved in current density or power density by optimizing the concentration of an electron mediator which is immobilized on a cathode or an anode together with an enzyme, a method for producing the same, an enzyme-immobilized electrode suitable for use as a cathode or anode of the biofuel cell, and a method for producing the electrode.

Another problem to be solved by the invention is to provide an electronic apparatus using the excellent biofuel cell.

The above-mentioned problems and other problems will be made clear from the description of the specification.

SUMMARY

The intensive research performed by the inventors for solving the problems resulted in the achievement of the present invention.

As far as the inventors known, there has been substantially no investigation of a method for increasing the concentration of an electron mediator immobilized together with an enzyme on a cathode or anode of a biofuel cell, moving the mediator in a fixed layer by diffusion, and maintaining high enzyme activity. However, according to the research conducted by the inventors, it has been found that these matters greatly influence the current density and the maintenance ratio thereof in a biofuel cell. In addition, as a result of the experimental and theoretical intensive research for optimizing the concentration of an electron mediator in a catalyst layer immobilized on an electrode, the inventors have reached the conclusion that it is effective to control the concentration of the electron mediator to be at least 10 times a Michaelis constant $K_m$ (Michaelis constant $K_m$ of enzymatic reaction of the electron mediator used as a substrate) of the electron mediator for an enzyme, which is determined by measurement in a solution, leading to the achievement of the present invention. When the concentration of the electron mediator in the catalyst layer immobilized on the electrode is at least 10 times the constant $K_m$, the current value reaches substantially the maximum.

That is, in order to resolve the problems, a first aspect of the present invention relates to a biofuel cell having a structure in which a cathode and an anode are opposed to each other with a proton conductor provided therebetween; wherein at least one of the cathode and the anode includes an electrode on which at least one enzyme and at least one electron mediator are immobilized; and

the concentration of the electron mediator immobilized on the electrode is at least 10 times a Michaelis constant $K_m$ of the electron mediator for the enzyme, which is determined by measurement in a solution.

In the biofuel cell, the electron mediator can be smoothly moved by diffusion in an immobilized layer, and the activity of the enzyme immobilized together with the electron mediator can be kept high.

A second aspect of the present invention relates to a method for producing a biofuel cell having a structure in which a cathode and an anode are opposed to each other with a proton conductor provided therebetween, and at least one of the cathode and the anode includes an electrode on which at least one enzyme and at least one electron mediator are immobilized, the method including controlling the concentration of the electron mediator immobilized on the electrode to be at least 10 times a Michaelis constant $K_m$ of the electron mediator for the enzyme, which is determined by measurement in a solution.

A third aspect of the present invention relates to an electron apparatus including at least one biofuel cell having a structure in which a cathode and an anode are opposed to each other with a proton conductor provided therebetween, wherein at least one of the cathode and the anode including an electrode on which at least one enzyme and at least one electron mediator are immobilized, and

the concentration of the electron mediator immobilized on the electrode being at least 10 times a Michaelis constant $K_m$ of the electron mediator for the enzyme, which is determined by measurement in a solution.

As a material of the electrode of the cathode or anode, a carbon porous material is generally used, and examples of such a material which is frequently used include porous carbon, carbon pellets, carbon felt, carbon fibers, carbon paper, and the like. In particular, carbon felt is preferred from the viewpoint of its large surface area and easy handleability.

A material other than the carbon porous material may be used as the material of the electrode of the cathode or anode.

When the cathode and anode electrodes are carbon porous electrodes, there is an optimum range of each of the ratio of thickness and the ratio of surface area (nitrogen ($N_2$) gas-adsorbing surface area) between the cathode and anode electrodes. Each of the ratios is preferably 1 : 1 to 3 and more preferably 1 : 2 to 2.5. For example, the ratio of thickness between the cathode and anode electrodes is 1 : 2.33, and the ratio of surface area between the cathode and anode electrodes is 1 : 2. In addition, particularly, there is an optimum range of the ratio between the volume of the anode electrode (including void) and the volume of the enzyme/electron mediator immobilized layer of the anode. This ratio is 50 to 30 : 1. For example, the ratio is 40.3 : 1.

The enzyme immobilized on the cathode and anode electrodes may be any one of various types and is appropriately selected according to the fuel used.

For example, when a monosaccharide such as glucose is used as the fuel, the enzyme immobilized on the anode electrode contains an oxidase which accelerates oxidation of the monosaccharide to decompose it, and generally further contains a coenzyme oxidase which returns a coenzyme reduced with the oxidase to an oxidized form. When the coenzyme is returned to the oxidized form by the action of the coenzyme oxidase, electrons are produced and transferred to the electrode from the coenzyme oxidase through the electron mediator. For example, $NAD^+$-dependent glucose dehydrogenase (GDH) is used as the oxidase, nicotinamide adenine dinucleotide ($NAD^+$) is used as the coenzyme, and diaphorase (DI) is used as the coenzyme oxidase.

When a polysaccharide (polysaccharide in a board sense representing all carbohydrates which produce two or more molecules of monosaccharides by hydrolysis and including oligosaccharides such as disaccharides, trisaccharides, tetrasaccharides, and the like) is used as the fuel, preferably, a catabolic enzyme which accelerates hydrolysis of the polysaccharide to produce a monosaccharide such as glucose is also immobilized in addition to the oxidase, the coenzyme oxidase, the coenzyme, and the electron mediator. Examples of the polysaccharide include starch, amylose, amylopectin, glycogen, cellulose, maltose, sucrose, lactose, and the like. Any one of these polysaccharides contains bonds of two or more monosaccharides and glucose as a bonding unit monosaccharide. In addition, amylose and amylopectin are components contained in starch, and starch is composed of a mixture of amylose and amylopectin. When glycoamylase and glucose dehydrogenase are used as the catabolic enzyme of a polysaccharide and the oxidase which degrades a monosaccharide, respectively, electric power can be generated using the polysaccharide as the fuel as long as the fuel contains any one of polysaccharides capable of degrading to glucose with the glycoamylase, for example, starch, amylose, amylopectin, glycogen, and maltose. The glycoamylase is a catabolic enzyme which hydrolyzes $\alpha$-glucan such as starch to produce glucose, and the glucose dehydrogenase is an oxidase which oxidizes $\beta$-D-glucose to D-glucono-$\delta$-lactone. In a preferred configuration, the catabolic enzyme which degrades the polysaccharide is also immobilized on the anode, and the polysaccharide finally used as the fuel is also immobilized on the anode.

When starch is used as the fuel, starch may be gelatinized and used as gelled solid fuel. In this case, preferably, it is possible to use a method of bringing the gelatinized starch into contact with the anode on which the enzyme is immobilized or immobilizing the starch on the anode together with the enzyme. By using such an electrode, the starch concentration on the surface of the anode can be kept higher than that in use of starch dissolved in a solution, thereby accelerating the degradation reaction with the enzyme and increasing power. Further, the fuel can be easily handled as compared with the solution, and thus a fuel supply system can be simplified. Further, the need for "do not turn over the biofuel cell" can be eliminated, and thus the biofuel cell is very advantageous for use in, for example, a mobile apparatus.

When methanol is used as the fuel, methanol is degraded to $CO_2$ through a three-step oxidation process using, as catalysts, alcohol dehydrogenase (ADH) which acts on methanol to oxidize it to formaldehyde, formaldehyde dehydrogenase (FalDH) which acts on formaldehyde to oxidize it to formic acid, and formate dehydrogenase which acts on formic acid to oxidize it to $CO_2$. Namely, three molecules of NADH per molecule of methanol are produced, producing six electrons.

When ethanol is used as fuel, ethanol is degraded to acetic acid through a two-step oxidation process using, as catalysts, alcohol dehydrogenase (ADH) which acts on ethanol to oxidize it to acetaldehyde and acetaldehyde dehydrogenase (AIDH) which acts on acetaldehyde to oxidize it to acetic acid. Namely, a total of four electrons per molecule of ethanol is produced through the two-step oxidation process.

A method of degrading ethanol into $CO_2$ by the same process as that for methanol can be employed. In this case, acetaldehyde dehydrogenase (AalDH) is caused to act on acetaldehyde to produce acetyl CoA which is then transferred to a TCA circuit. The TCA circuit further produces electrons.

As the electron mediator immobilized on the anode electrode together with the enzyme, basically, any mediator may be used, but a quinone-type electron mediator, more specifically a compound having a quinone skeleton, particularly a compound having a naphthoquinone skeleton, is preferably used. As the compound having a naphthoquinone skeleton, various naphthoquinone derivatives can be used. Examples of the derivatives include 2-amino-1,4-naphthoquinone (ANQ), 2-amino-3-methyl-1,4-naphthoquinone (AMNQ), 2-methyl-1,4-naphthoquinone ($VK_3$), 2-amino-3-carboxy-1,4-naphthoquinone (ACNQ), and the like. As the compound having a quinone skeleton, for example, anthraquinone and its derivatives other than the compounds having a naphthoquinone skeleton can be used. Besides the compound having a quinone skeleton, the electron mediator may contain at least one other compound acting as an electron mediator according to demand.

When 2-methyl-1,4-naphthoquinone (VK$_3$) is used as the electron mediator, the concentration of the electron mediator immobilized on the electrode is preferably 4 $\mu$M or more. When 2-amino-3-carboxy-1,4-naphthoquinone (ACNQ) is used as the electron mediator, the concentration of the electron mediator immobilized on the electrode is preferably 20 $\mu$M or more. When 2-amino-1,4-naphthoquinone (ANQ) is used as the electron mediator, the concentration of the electron mediator immobilized on the electrode is preferably 360 $\mu$M or more.

The enzyme immobilized on the cathode electrode typically contains an oxygen reductase. As the oxygen reductase, for example, bilirubin oxidase, laccase, ascorbate oxidase, and the like can be used. Examples of the electron mediator immobilized on the cathode electrode in addition to the enzyme include potassium hexacyanoferrate (K$_3$[Fe (CN)$_6$]), potassium ferricyanide, potassium octacyanotungstate, and the like.

An immobilizing material for immobilizing the enzyme, the coenzyme, and the electron mediator on the anode and cathode electrodes, various materials can be used. For example, a polyion complex formed using a polycation or its salt, such as poly-L-lycine (PLL), and a polyanion or its salt, such as polyacrylic acid (e.g., sodium polyacrylate (PAAcNa)), a combination of glutaraldehyde (GA) and poly-L-lycine (PLL), and other polymers may be used.

As the proton conductor (separator), any one of various conductors can be used and selected according to demand. Examples of the proton conductor include cellophane, nonwoven fabrics, perfluorocarbonsulfonate (PFS) resin films, trifluorostyrene derivative copolymer films, polybenzimidazole films impregnated with phosphoric acid, aromatic polyetherketone sulfonic acid films, PSSA-PVA (polystyrenesulfonic acid-polyvinyl alcohol) copolymers, PSSA-EVOH (polystyrenesulfonic acid-ethylene vinyl alcohol) copolymers, ion-exchange resin having fluorine-containing carbonsulfonic acid groups (Nafion (trade name, U.S. DuPont)), and the like.

For example, cellophane is used as the proton conductor (separator), and the enzyme and the electron mediator are immobilized on the cathode composed of a porous material such as carbon so that oxygen can be taken in from the air, thereby improving the oxygen supply to the electrode and significantly improving the current value (refer to Patent Document 6).

When an electrolyte containing a buffer material (buffer solution) is used as the proton conductor, in order that even when the amount of protons is increased or decreased in the electrode or the enzyme-immobilized layer by enzymatic reaction through the protons in a high-output operation, a sufficient buffer function can be achieved, a deviation of pH from proper pH can be sufficiently suppressed, and the ability inherent in the enzyme can be sufficiently exhibited, it is effective to control the concentration of the buffer material contained in the electrolyte to 0.2 M to 2.5 M. The concentration is preferably 0.2 M to 2 M, more preferably 0.4 M to 2 M, and further preferably 0.8 M to 1.2 M. As the buffer material, any material may be used as long as pKa is 5 to 9. Examples of the buffer material include dihydrogen phosphate ion (H$_2$PO$_4$$^-$), 2-amino-2-hydroxymethyl-1,3-propanediol (abbreviated as "Tris"), 2-(N-morpholino)ethanesulfonic acid (MES), cacodylic aid, carbonic acid (H$_2$CO$_3$), hydrogen citrate ion, N-(2-acetamido)iminodiacetic acid (ADA), piperazine-N,N'-bis(2-ethanesulfonic acid)(PIPES), N-(2-acetamido)-2-aminoethanesulfonic acid (ACES), 3-(N-morpholino)propanesulfonic acid (MOPS), N-2-hydroxyethylpiperazine-N'-2-ethanesulfonic acid (HEPES), N-2-hydroxyethylpiperazine-N'-3-propanesulfonic acid (HEPPS), N-[tris(hydroxymethyl)methyl]glycine (abbreviated as "Tricine"), glycylglycine, N,N-bis(2-hydroxyethyl)glycine (abbreviated as "Bicine"), and the like. Examples of materials which produce dihydrogen phosphate ion (H$_2$PO$_4$$^-$) include sodium dihydrogen phosphate (NaH$_2$PO$_4$), potassium dihydrogen phosphate (KH$_2$PO$_4$), and the like. As the buffer material, a compound containing an imidazole ring is also preferred. Examples of the compound containing an imidazole ring include imidazole, triazole, pyridine derivatives, bipyridine derivatives, imidazole derivatives (histidine, 1-methylimidazole, 2-methylimidazole, 4-methylimidazole, 2-ethylimidazole, ethyl imidazole-2-carboxylate, imidazole-2-carboxyaldehyde, imidazole-4-carbaxylic acid, imidazole-4,5-dicarboxylic acid, imidazol-1-yl-acetic acid, 2-acetylbenzimidazole, 1-acetylimidazole, N-acetylimidazole, 2-aminobenzimidazole, N-(3-aminopropyl)imidazole, 5-amino-2-(trifluoromethyl)benzimidazole, 4-azabenzimidazole, 4-aza-2-mercaptobenzimidazole, benzimidazole, 1-benzylimidazole, 1-butylzmidazole), and the like. As the buffer material, 2-aminoethanol, triethanolamine, TES (N-Tris(hydroxymethyl)methyl-2-aminoethanesulfonic acid), BES (N,N-bis(2-hydroxyethyl)-2-aminoethanesulfonic acid), and the like may be used. The pH of the electrolyte containing the buffer material is preferably about 7, but generally may be any value of 1 to 14. If required, the buffer material may be immobilized on the layer in which the enzyme and the electron mediator are immobilized.

The biofuel cell can be used for all devices requiring electric power regardless of size. For example, the biofuel cell can be used for electronic apparatuses, mobile bodies (bicycles, two-wheel vehicles, aircrafts, rockets, spacecrafts, and the like), power plants, construction machines, machine tools, power generation systems, cogeneration systems, and the like. The power, size, shape, and fuel type are determined according to application.

Electronic apparatuses may be basically any apparatuses and include a portable type and a stationary type. Examples of the electronic apparatuses include cellular phones, mobile devices, robots, personal computers, game devices, automobile-installed equipment, home electric appliances, industrial products, and the like.

A fourth aspect of the present invention relates to an enzyme-immobilized electrode including an electrode on which at lease one enzyme and at lease one electron mediator are immobilized, wherein the concentration of the electron mediator immobilized on the electrode is at least 10 times a Michaelis constant $K_m$ of the electron mediator for the enzyme, which is determined by measurement in a solution.

A fifth aspect of the present invention relates to a method for producing an enzyme-immobilized electrode including an electrode on which at lease one enzyme and at lease one electron mediator are immobilized, the method including controlling the concentration of the electron mediator immobilized on the electrode to be at least 10 times a Michaelis constant $K_m$ of the electron mediator for the enzyme, which is determined by measurement in a solution.

In the fourth and fifth aspects of the present invention, besides the above-described matters, the matters described with respect to the first and second aspects of the present invention are established as long as they are not contrary to the properties.

In the present invention having the above-described constitution, the concentration of the electron mediator immobilized together with the enzyme on the electrode of the cathode or the anode is as high as at least 10 times a Michaelis constant $K_m$ of the electron mediator for the enzyme, which is determined by measurement in a solution, and thus the electron mediator can easily moves in the immobilized layer by diffusion while staying in the immobilized layer, thereby permitting rapid electron transfer reaction between the enzyme and the electrode through the electron mediator.

According to the present invention, it is possible to realize a biofuel cell in which the concentration of an electron mediator immobilized together with an enzyme on an anode or cathode electrode is optimized so that the current density or power density can be significantly improved. In addition, a high-performance electronic apparatus and the like can be realized using the excellent biofuel cell as a power supply.

BRIEF DESCRIPTION OF THE DRAWINGS

Fig. 1 is a schematic diagram showing a biofuel cell according to a first embodiment of the present invention.

Fig. 2 is a schematic diagram schematically showing detailed configurations of an anode and a cathode and electron transfer reactions on the anode and the cathode of the biofuel cell according to the first embodiment of the present invention.

Fig. 3 is a photograph, as an alternative to a drawing, showing a confocal microscope image of an immobilized layer formed in the first embodiment of the present invention.

Fig. 4 is a schematic diagram showing an electrochemical measurement cell using a GC bio-anode prepared for evaluating the anode of the biofuel cell according to the first embodiment of the present invention.

Fig. 5 is a graph showing the results of cyclic voltammetry performed using the electrochemical measurement cell shown in Fig. 4.

Fig. 6 is a schematic diagram showing an electrochemical measurement cell using a CF bio-anode prepared for evaluating the anode of the biofuel cell according to the first embodiment of the present invention.

Fig. 7 is a graph showing the dependency of current density on the buffer solution concentration on the basis of the results of chronoamperometry performed using the electrochemical measurement cell shown in Fig. 6.

Fig. 8 is a graph showing changes of pH in the CF bio-anode used in chronoamperometry, the CF bio-anode being prepared for evaluating the anode of the biofuel cell according to the first embodiment of the present invention.

Fig. 9 is a graph showing the dependency of relative current on initial pH of a phosphate buffer bulk solution in the CF bio-anode used in chronoamperometry, the CF bio-anode being prepared for evaluating the anode of the biofuel cell according to the first embodiment of the present invention.

Fig. 10 is a graph showing the dependency of relative activity of GDH and DI on the buffer solution concentration on the basis of an experiment performed for evaluating the anode of the biofuel cell according to the first embodiment of the present invention.

Fig. 11 is a graph showing the results of cyclic voltammetry performed by changing the sweep speed using the electrochemical measurement cell shown in Fig. 4.

Fig. 12 is a graph showing the results of cyclic voltammetry performed by changing the ANQ concentration in a solution containing sufficient amounts of NADH and DI.

Fig. 13 is a graph showing the dependency of current on the ANQ concentration on the basis of the results of cyclic voltammetry shown in Fig. 12.

Fig. 14 is a graph showing the results of cyclic voltammetry using $VK_3$, ACNQ, and ANQ in a solution containing sufficient amounts of NADH and DI.

Fig. 15 is a graph showing the dependency of current on the concentrations of $VK_3$, ACNQ, and ANQ on the basis of the results of cyclic voltammetry shown in Fig. 14.

Fig. 16 is a schematic diagram showing a sink-type electrochemical measurement cell using a CF bio-cathode prepared for evaluating the cathode of the biofuel cell according to the first embodiment of the present invention.

Fig. 17 is a schematic diagram showing an open-air-type electrochemical measurement cell using a CF bio-cathode prepared for evaluating the cathode of the biofuel cell according to the first embodiment of the present invention.

Fig. 18 is a graph showing the results of chronoamperometry performed using the sink-type electrochemical measurement cell shown in Fig. 16 and the open-air-type electrochemical measurement cell shown in Fig. 17.

Fig. 19 is a graph showing the results of measurement of the water content in a CF bio-cathode prepared for evaluating the cathode of the biofuel cell according to the first embodiment of the present invention.

Fig. 20 is a graph showing changes of pH in the CF bio-cathode used in chronoamperometry, the CF bio-cathode being prepared for evaluating the cathode of the biofuel cell according to the first embodiment of the present invention.

Fig. 21 is a graph showing the dependency of relative current on initial pH of a phosphate buffer bulk solution in the CF bio-cathode used in chronoamperometry, the CF bio-cathode being prepared for evaluating the cathode of the biofuel cell according to the first embodiment of the present invention.

Fig. 22 is a graph showing the dependency of relative current on the buffer solution concentration on the basis of experiment performed using a GC electrode in a solution containing BOD and $K_3[Fe(CN)_6]$ for evaluating the cathode of the biofuel cell according to the first embodiment of the present invention.

Fig. 23 is a schematic diagram showing an electrochemical measurement cell prepared for evaluating the biofuel cell according to the first embodiment of the present invention.

Fig. 24 is a graph showing the voltage characteristics of current and output measured using the electrochemical measurement cell shown in Fig. 23.

Fig. 25 is a graph showing the voltage characteristics of current of the anode during an operation of the electrochemical measurement cell shown in Fig. 23.

Fig. 26 is a graph showing the voltage characteristics of current of the cathode during operation of the electrochemical measurement cell shown in Fig. 23.

Fig. 27 is a sectional view showing a stacked biofuel cell unit according to a second embodiment of the present invention.

Fig. 28 is an exploded perspective view showing the stacked biofuel cell unit according to the second embodiment of the present invention.

Fig. 29 is a side view showing a fuel holding vessel of the stacked biofuel cell unit according to the second embodiment of the present invention.

Fig. 30 is a perspective view showing an example of a spacer of the stacked biofuel cell unit according to the second embodiment of the present invention.

DETAILED DESCRIPTION

Embodiments of the present invention will be described with reference to the drawings.

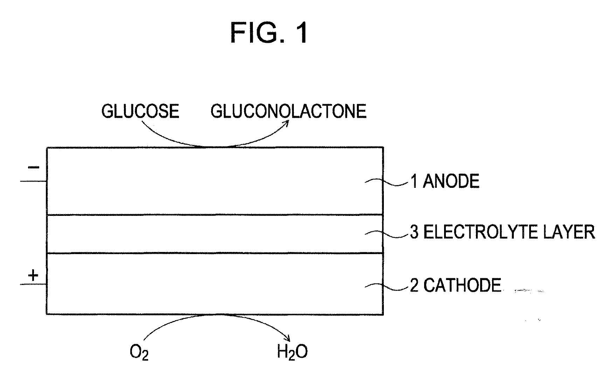

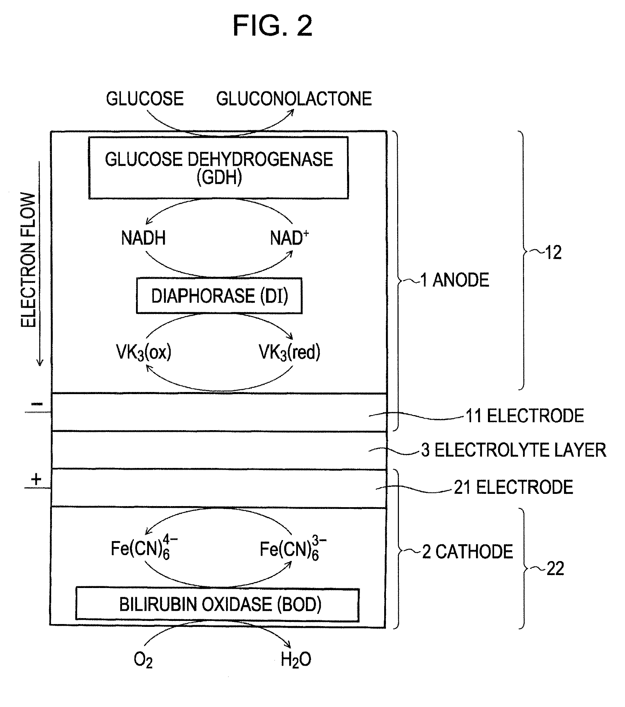

Fig. 1 schematically shows a biofuel cell according to a first embodiment of the present invention. The biofuel cell uses glucose as fuel. Fig. 2 schematically shows detailed configurations of an anode and a cathode and electron transfer reactions on the anode and the cathode of the biofuel cell.

As shown in Fig. 1, the biofuel cell has a structure in which an anode 1 and a cathode 2 are opposed to each other with an electrolyte layer 3 provided therebetween, the electrolyte layer 3 transferring only protons. On the anode 1, the glucose supplied as the fuel is degraded with an enzyme to take out electrons and produce protons ($H^+$). On the cathode 2, water is produced from the protons transported through the electrolyte layer, electrons supplied from the anode 1 through an external circuit, and oxygen ($O_2$), for example, in air.

The anode 1 has a configuration in which an enzyme involved in glucose degradation, a coenzyme (e.g., $NAD^+$, $NADP^+$, or the like) which produces a reduced form with an oxidation reaction in the glucose degradation process, a coenzyme oxidase (e.g., diaphorase (DI)) which oxidizes the reduced form (e.g., NADH, NADPH, or the like) of the coenzyme, and an electron mediator which receives the electrons produced from the coenzyme oxidase with oxidation of the coenzyme and supplies the electrons to an electrode 11 are immobilized on the electrode 11 with a predetermined immobilizing material, the electrode 11 being composed of, for example, a carbon porous material (refer to Fig. 2). Reference numeral 12 denotes an immobilized layer. In order to achieve a sufficient catalyst current density, it is important to densely immobilize the enzyme, the coenzyme, and the electron mediator on the anode 1 without decreasing the biological and electrochemical activity thereof.

As the enzyme involved in the degradation of glucose, for example, glucose dehydrogenase (GDH) can be used. In the presence of the oxidase, for example, β-D-glucose can be oxidized into D-glucono-δ-lactone.

Further, the D-glucono-δ-lactone can be degraded into 2-keto-6-phospho-D-gluconate in the presence of two enzymes, i.e., gluconokinase and phosphogluconate dehydrogenase (PhGDH). Namely, the D-glucono-δ-lactone is hydrolyzed to D-gluconate, and the D-glueonate is phosphorylated to 6-phospho-D-gluconate by hydrolysis of adenosine triphosphate (ATP) into adenosine diphosphate (ADP) and phosphoric acid in the presence of gluconokinase. The 6-phospho-D-gluconate is oxidized to 2-keto-6-phospho-D-gluconate by the action of the oxidase PhGDH.

In addition, besides the above-described degradation process, glucose can be degraded into $CO_2$ using glycometabolism. The degradation process using the glycometabolism is roughly divided into degradation of glucose by a glycolytic system, production of pyruvic acid, and the TCA circuit. These are widely known reaction systems.

An oxidation reaction in the degradation process of a monosaccharide proceeds accompanying a reduction reaction of a coenzyme. The coenzyme substantially depends on the enzyme on which the coenzyme acts, and $NAD^+$ is used as the coenzyme of GDH. That is, when β-D-glucose is oxidized into D-glucono-δ-lactone by the action of GDH, $NAD^+$ is reduced to NADH to produce H+.

The produced NADH is immediately oxidized into $NAD^+$ in the presence of diaphorase (DI), producing two electrons and $H^+$. Therefore, two electrons and two $H^+$ per molecule of glucose are produced in an one-step oxidation reaction. A two-step oxidation reaction produces a total of four electrons and four $H^+$.

The electrons produced in the above-described process are transferred to the electrode 11 from diaphorase through the electron mediator, and $H^+$ are transported to the cathode 2 through the electrolyte layer 3.

The electron mediator transfers the electrons from and to the electrode 11, and the output voltage of the biofuel cell depends on the oxidation-reduction potential of the electron mediator. In other words, an electron mediator with more negative potential may be selected on the anode 1 side for achieving a higher output voltage, but it is necessary to take into consideration the reaction affinity of the electron mediator for the enzyme, the electron exchange rate with the electrode 11, and structural stability for inhibitors (light, oxygen, and the like). From this viewpoint, as the electron mediator immobilized on the electrode 11 of the anode 1 together with the enzyme and the coenzyme, a quinone-type mediator, particularly an electron mediator having a naphthoquinone skeleton, is preferably used. Examples of such an electron mediator include 2-methyl-1,4-naphthoquinone ($VK_3$), 2-amino-3-carboxy-1,4-naphthoquinone (ANQ), 2-amino-1,4-naphthoquinone (ANQ), and the like.

The concentration of the electron mediator immobilized on the electrode 11 is selected to be at least 10 times the Michaelis constant $K_m$ of the electron mediator for the enzyme, particularly the coenzyme oxidase, immobilized on the electrode 11, the constant being determined by measurement in a solution. For example, when diaphorase is used as the coenzyme oxidase, the concentration of the electron mediator immobilized on the electrode 11 is selected to be 4 μM or more in use of $VK_3$ as the electron mediator, 20 μM or more in use of ACNQ as the electron mediator, and 360 μM or more in use of ANQ as the electron mediator (refer to Table 1 described below).

The electrolyte layer 3 serves as the proton conductor which transports $H^+$ produced on the anode 1 to the cathode 2 and is composed of a material capable of transporting $H^+$ without having electron conductivity. For example, the electrolyte layer 3 can be appropriately selected from the above-described materials and used. In this case, the electrolyte layer 3 contains the buffer solution containing as the buffer material, for example, dihydrogen phosphate ion ($H_2PO_4^-$), sodium dihydrogen phosphate ($NaH_2PO_4$), or a compound having an imidazole ring. The concentration of the buffer material is selected according to demand but is preferably 0.2 M to 2 M. In this case, high buffer ability can be achieved and the ability inherent in the enzyme can be sufficiently exhibited in a high-output operation of the biofuel cell. Further, excessively high or low ion strength (I. S.) adversely affects the enzyme activity, but in view of electrochemical response, the ion strength is preferably a proper value, for example, about 0.3. However, the optimum values of pH and ion strength depend on the enzyme used and are not limited to the above-described values. The buffer solution permeates into not only the electrolyte layer 3 but also the anode 1 and the cathode 2.

As an immobilizing material for immobilizing the enzyme, the coenzyme, and the electron mediator on the electrode 11, for example, a polyion complex prepared by combining sodium polyacrylate (PAAcNa) and poly-L-lycine (PLL) is preferably used, but the material is not limited to this.

The cathode 2 includes an electrode 21 composed of a material having voids therein, particularly a carbon porous material, e.g., carbon felt or porous carbon, and an oxygen reductase and an electron mediator which are immobilized on the electrode 21, the electron mediator transferring electrons to and from the electrode 21. Reference numeral 22 denotes an immobilized layer. As the oxygen reductase, for example, bilirubin oxidase (BOD), laccase, ascorbate oxidase, and the like can be used. As the electron mediator, for example, hexacyanoferrate ion $Fe(CN)_6^{4-/3-}$ produced by ionization of potassium hexacyanoferrate $K_3[Fe(CN)_6]$ can be used.

On the cathode 2, oxygen in air is reduced with $H^+$ from the electrolyte layer 3 and electrons from the anode 1 in the presence of the oxygen reductase to produce water.

Fig. 2 shows an example in which on the anode 1, the enzyme involved in glucose degradation is glucose dehydrogenase (GDH), the coenzyme which produces a reduced form with an oxidation reaction in the glucose degradation process is $NAD^+$, the coenzyme oxidase which oxidizes the reduced form NADH of the coenzyme is diaphorase (DI), and the electron mediator which receives electrons from the coenzyme oxidase with oxidation of the coenzyme and transfers the electrons to the electrode 11 is $VK_3$, while on the cathode 2, the oxygen reductase is bilirubin oxidase (BOD) and the electron mediator which receives electrons from the electrode 21 and transfers the electrons to the oxygen reductase is $Fe(CN)_6^{4-/3-}$.

In an operation (during use) of the biofuel cell configured as described above, when glucose is supplied to the anode 1 side, the glucose is degraded with the catabolic enzyme including the oxidase. Since the oxidase is involved in the degradation process of the monosaccharide, electrons and $H^+$ can be produced on the anode 1 side, and a current can be generated between the anode 1 and the cathode 2. In order to take out the current to the outside from the biofuel cell, for example, current collectors (not shown) such Ti meshes are respectively provided between the electrode 11 and the electrolyte layer 3 and between the electrode 21 and the electrolyte layer 3.

The results of experiments conducted for evaluating the anode 1 of the biofuel cell will be described.

The surface of a glassy carbon (GC) electrode of 3 mm in diameter which was purchased from BAS Inc. was polished with alumina powder having a particle diameter of 0.05 $\mu$m and washed with distilled water. Then, GDH, NADH, DI, and $VK_3$ were immobilized on the GC electrode by a polyion complex method using electrostatic interaction between PAAcNa as polyanion and poly-L-lycine (PLL) as polycation according to the following procedures.

On the GC electrode, 10 $\mu$l of an aqueous PLL solution (2% (w/v)), 8 $\mu$l of a GDH solution (1 U/$\mu$l in a 0.1 M phosphate buffer solution ($NaH_2PO_4$ buffer solution) (pH 7.0)), 8 $\mu$l of a DI solution (10 U/$\mu$l in a phosphate buffer solution), 2 $\mu$l of a NADH solution (0.9 $\mu$mol/$\mu$l in a phosphate buffer solution), 4 $\mu$l of a $VK_3$ solution (0.29 M in acetone), and 4 $\mu$l of an aqueous PAAcNa solution (0.066% (w/v)) were added in that order. After each of the addition steps, drying was performed at 40°C for 10 minutes. Before electrochemical measurement, the GC electrode (referred to as a "GC bio-anode" hereinafter) on which an immobilized layer had been formed as described above was rinsed with distilled water for 5 minutes.

GDH (EC1. 1. 1. 47) derived from Bacillus sp. was used as GDH, and DI(EC. 1. 6. 99. -) derived from Bacillus stearothermophilus was used as DI. Both were purchased from Amano Enzyme Inc. (Japan) and directly used without further purification. NADH, poly-L-lycine (PLL, Mw = about 93,800), and sodium polyacrylate (PAANa, Mw = about 240,000) were purchased from Sigma-Aldrich Co. $VK_3$ was purchased from Nacalai Tesque, Inc. (Japan).

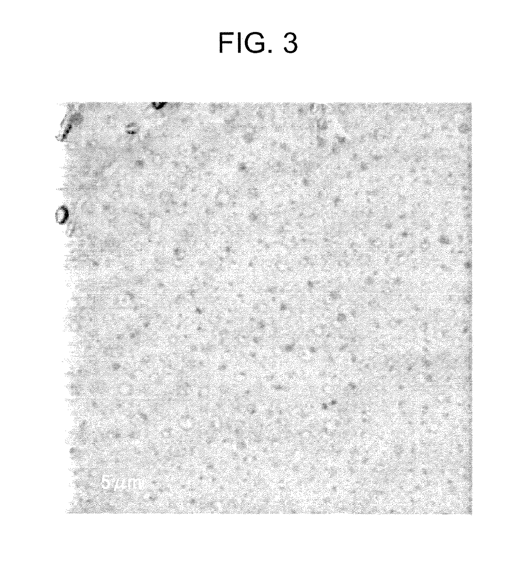

As a result of measurement of the thickness of the immobilized layer with a confocal microscope (FV1000-BX6, Olympus Co. Japan) before the electrochemical measurement, the dry thickness was 53 $\mu$m. Fig. 3 shows a confocal microscope image (actually a color image). In the confocal microscope image, orange portions corresponding to fluorescence of $VK_3$ are observed. A crater-like structure is possibly produced in the drying step. The thickness of the immobilized layer was determined from a difference in focal point between the uppermost surface of the immobilized layer and the surface of the GC electrode.

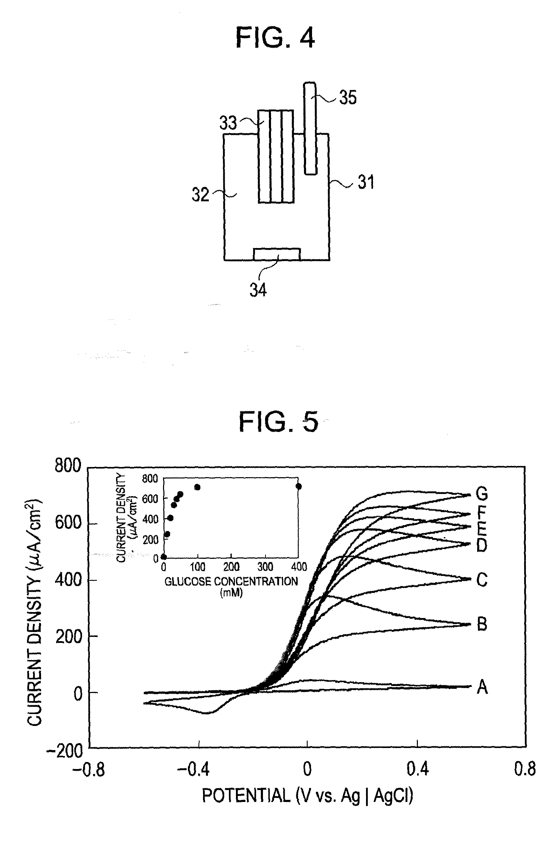

Cyclic voltammetry (CV) was performed using the GC bio-anode as a working electrode. The cyclic voltammetry was performed using 1480 Multi-Stat (Solarton Analytical) at room temperature in a three-electrode system without convection in a measurement solution. Fig. 4 shows an electrochemical measurement cell used in an experiment. As shown in Fig. 4, a measurement solution (electrolyte solution) 32 was placed in a vessel 31, and the GC bio-anode as a working electrode 33, a Pt plate as a counter electrode 34, and an Ag|AgCl|saturated KCl electrode as a reference electrode 35 were immersed in the measurement solution 32. An electrochemical measurement device (not shown) was connected to the working electrode 33, the counter electrode 34, and the reference electrode 35. The counter electrode 34 was set at the bottom of the vessel 31. The volume of the measurement solution 32 was 1 ml. A 0.1 M phosphate buffer solution (pH 7.0) containing each of 0 mM (A), 10 mM (B), 20 mM (C), 30 mM (D), 40 mM (E), 50 mM (F), and 100 mM (G) of glucose was used as the measurement solution 32. Before the measurement, $O_2$ in the buffer solution was removed by bubbling with Ar gas saturated with $H_2O$.

Fig. 5 shows the results of cyclic voltammetry. A graph inserted into Fig. 5 shows the dependency of the glucose concentration on the stationary current value of the cyclic voltammetry. As shown in Fig. 5, when glucose is absent from the measurement solution 32, a pair of oxidation and reduction waves of immobilized $VK_3$ having a middle point potential of -0.17 V vs. Ag|AgCl is obtained (wave A in Fig. 5). When glucose is present in the measurement solution 32, catalytic waves of glucose oxidation appear (waves B to G in Fig. 5), and the current increases as the glucose concentration increases. When the glucose concentration is 20 mM or more, the GC bio-anode shows a stationary wave with the maximum current density of 0.7 mA/cm² at 0.4 M glucose. In this case, the reaction rate possibly depends on the enzymatic reaction. The waveform is constant during a plurality of times of sweep of CV. This indicates that the four components, e.g., GDH, NADH, DI, and $VK_3$, are stably and densely immobilized in the immobilized layer while maintaining GDH and DI enzyme activity.

Further, in order to increase the current density, a carbon fiber (CF) sheet which is preferred because it has large surface area and does not inhibit glucose transport was used as the electrode 11 instead of GC, and GDH, NADH, DI, and $VK_3$ were immobilized by the same method as the GC bio-anode. Specifically, each of the solutions used for preparing the GC bio-anode was added in a volume of 14 times the volume for the GC bio-anode to a laminate of four CF sheets and dried under the same conditions as for the GC bio-anode. Hereinafter, the CF sheet having the immobilized layer formed therein is referred to as a "CF bio-anode". As the CF sheet, a CF sheet (B0050, 750 $\mu$m thickness) purchased from Toray Industries, Inc (Japan) was used and cut into a 10 mm square. The BET specific surface area of the CF sheet after degassing at 200°C for 2 hours was 0.17 m²/g according to nitrogen adsorption-desorption isothermal analysis at 77 K using Belsorp-Max apparatus (BEL Japan Inc., Japan). The total surface area of the laminate of the four 10-mm square CF sheets was 20.4 cm². In order to improve adsorption of the immobilized layer on the CF sheets, the CF sheets were treated with ozone using an ozone treatment apparatus (UV. TC. 110 (BIOFORCE Nanosciences. Inc.)) before immobilization of GDH, NADH, DI, and $VK_3$. The thickness of the CF bio-anode before the electrochemical measurement was 2.11 mm. The thickness was measured using a coolant-proof micrometer (Series No. 293, Mitutoyo Corporation, Japan) under a pressure of 26.5 $N/cm^2$.

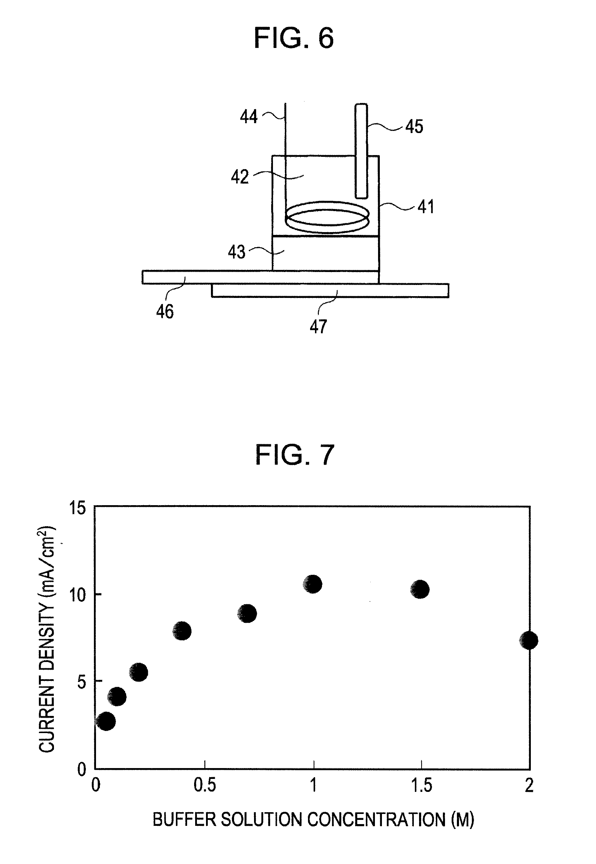

Chronoamperometry (CA) was performed using the CF bio-anode as a working electrode. The chronoamperometry was performed using 1480 Multi-Stat (Solarton Analytical) at room temperature in a three-electrode system without convection in a measurement solution. Fig. 6 shows an electrochemical measurement cell used in an experiment. As shown in Fig. 6, a measurement solution (electrolyte solution) 42 was placed in a vessel 41, and the CF bio-anode as a working electrode 43, a Pt wire as a counter electrode 44, and an Ag|AgCl|saturated KCl electrode as a reference electrode 45 were immersed in the measurement solution 42. An electrochemical measurement device (not shown) was connected to the working electrode 43, the counter electrode 44, and the reference electrode 45. The CF bio-anode as the working electrode 43 was set at the bottom of the vessel 41. Further, a Ti mesh was used as a current collector 46 for electric contact with the CF bio-anode used as the working electrode 43. The Ti mesh was purchased from Thank-Metal Co. (Japan). In addition, a cellophane film 47 was attached as a separator to the current collector 46. The cellophane film 47 was purchased from Futamura Chemical Co., Ltd. (Japan). The volume of the measurement solution 32 was 3 ml. A 0.1 M phosphate buffer solution (pH 7.0) containing of 0.4 M of glucose was used as the measurement solution 42. Before the measurement, $O_2$ in the buffer solution was removed by bubbling with Ar gas saturated with $H_2O$. The catalytic current density after the chronoamperometry at 0.1 V for 1 minute was only 5.9 times the catalytic current density on the GC bio-anode in spite of the surface area of the CF bio-anode estimated to be about 20 times that of the GC electrode. It was supposed from this result that the CF bio-anode has a low catalytic current density because of insufficient proton diffusion in the CF bio-anode. Therefore, it is thought that when proton diffusion in the CF bio-anode is insufficient, the proton concentration in the immobilized layer is increased due to glucose oxidation reaction (glucose $\rightarrow$ gluconolactone + $2H^+$ + $2e^-$), thereby decreasing the GDH and DI enzyme activity.

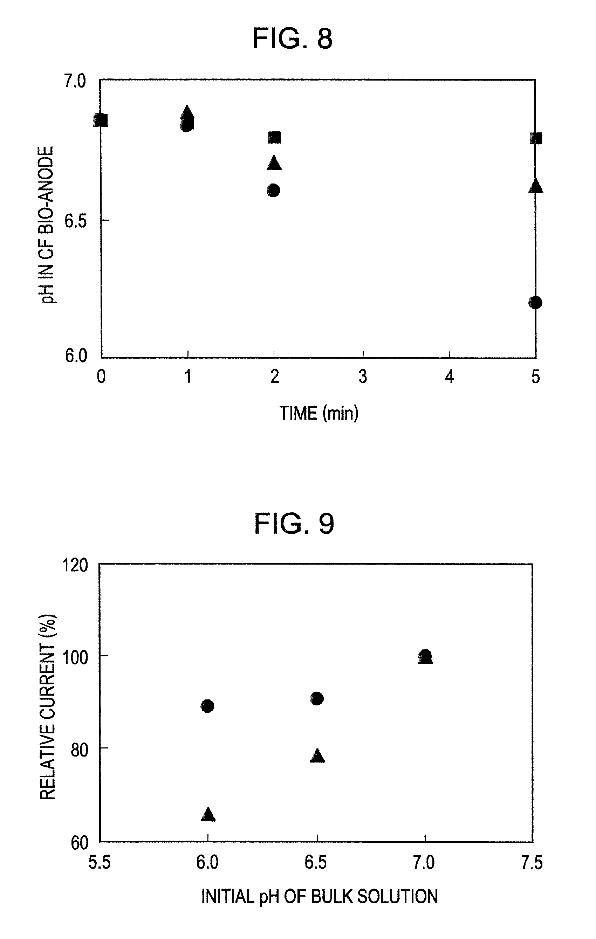

In order to resolve this problem, the inventors examined the effect of the buffer solution concentration in an electrolyte solution at a glucose concentration of 0.4 M. Fig. 7 shows the results of measurement of changes in the current density with the buffer solution concentration. The measurement was performed by the same method as described above using the electrochemical cell shown in Fig. 6. As shown in Fig. 7, the current density after chronoamperometry for 1 minute increases as the buffer solution concentration increases to 1.0 M. The current density reaches the maximum of 10.5 $mA/cm^2$ which is about 15 times the current density on the GC bio-anode at the buffer solution concentration of 1.0 M and then gradually decreases as the buffer solution concentration increases from 1.0 M. During the measurement, changes of pH of the solution in the CF bio-anode was monitored. The pH was measured with a pH meter (TYPE PCE308S-SR, Toko Chemical Laboratory Ltd., Japan) having a flat surface. The results of pH measurement are shown in Fig. 8. In Fig. 8, ● represents the buffer solution concentration of 0.1M, ▲ represents the buffer solution concentration of 1.0 M, and ■ represents the buffer solution concentration of 2.0 M. The results of pH measurement shown in Fig. 8 indicate that when the buffer solution concentration is 0.1 M, pH decreases from 6.85 to 6.20 within 5 minutes. This is significant as compared with a pH decrease (6.85 $\rightarrow$ 6.62) at the buffer solution concentration of 1.0 M or a pH decrease (6.85 $\rightarrow$ 6.79) at the buffer solution concentration of 2.0 M. As shown in Fig. 9, a decrease in pH causes a decrease in current density on the CF bio-anode due to a decrease in enzyme activity depending on pH. These results clearly indicate that a buffer solution at a higher concentration suppresses a decrease in pH in the CF bio-anode. In Fig. 9, ● represents the results after 1 minute and ▲ represents the results after 5 minutes.

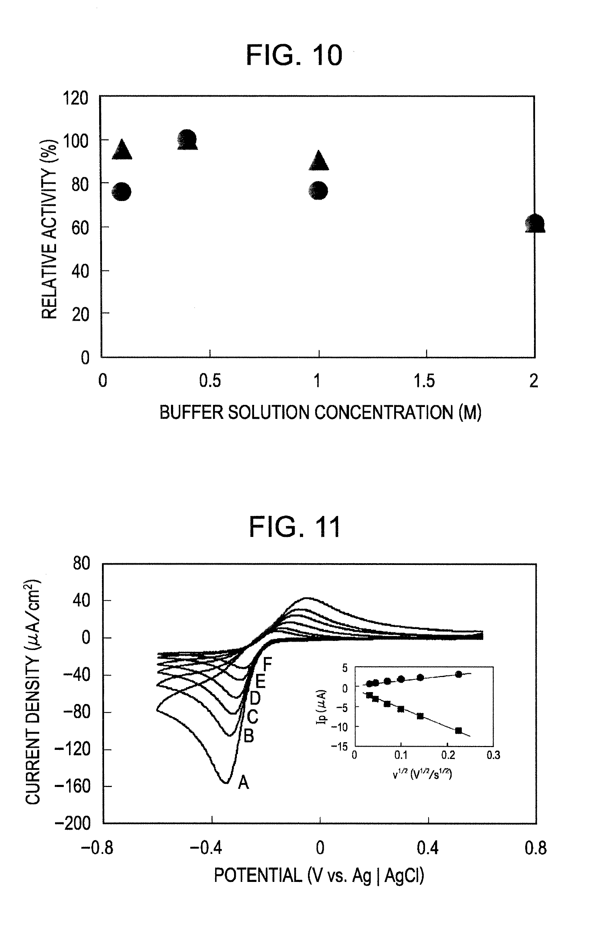

On the other hand, when the buffer solution concentration is as high as 1.0 to 2.0 M, the current density after chronoamperometry for 1 minute decreases. This is possibly due to the fact that as shown in Fig. 10, activity of each of GDH and DI in the solution decreases as the buffer solution concentration increases from 0.4 M to 2.0 M. In Fig. 10, ● represents GDH and ▲ represents DI. In Fig. 10, the enzyme activity of each of GDH and DI at the buffer solution concentration of 0.4 M is regarded as 100%. Therefore, it is found that there are two rate-determining processes, i.e., insufficient proton diffusion at a lower buffer solution concentration (< 1.0 M) and a decrease in enzyme activity at a higher buffer solution concentration (> 1.0 M). It is also found that the immobilized layer on the CF electrode is stable up to a buffer solution concentration of 2.0 M at maximum.

The confocal microscope image of the GC bio-anode shown in Fig. 2 suggests that a large amount of $VK_3$ is uniformly immobilized. Fig. 11 shows the results of cyclic voltammetry performed by changing the sweep speed v between potentials of -0.6 V and +0.6 V using the electrochemical measurement cell shown in Fig. 4. The sweep speed v was changed in the six steps of 50 mV/s (A), 20 mV/s (B), 10 mV/s (C), 5 mV/s (D), 2 mV/s (E), and 1 mV/s (F). As the measurement solution 32, a 0.1 M phosphate buffer solution (pH 7.0) not containing glucose was used. As shown in Fig. 11, an oxidation-reduction wave in the absence of glucose is due to an oxidation-reduction reaction of $VK_3$ immobilized on the GC bio-anode. The linear dependency of peak current on the square root $v^{1/2}$ of the weep speed v (a diagram inserted into Fig. 11) indicates that $VK_3$ can be diffused in the immobilized layer having a thickness of 53 $\mu m$ in a dry state. The $VK_3$ concentration in the immobilized layer determined by the Faraday electricity (2.8 $mC/cm^2$) calculated from oxidation of the $VK_3$ reduced form was 2.8 mM.

The electrochemical method of determining the $VK_3$ concentration in the immobilized layer is described in detail. The GC bio-anode was subjected to electrolytic reduction (-0.6 V vs. Ag|AgCl) for a sufficient time in a sufficiently deoxidized measurement solution (phosphate buffer solution) to perform constant-current measurement with a sufficiently more electropositive potential (0.6 V vs. Ag|AgCl) than the oxidation-reduction potential of $VK_3$. On the basis of the resultant current response curve with time, the $VK_3$ concentration C and diffusion constant D were determined by fitting the Cottrell equation below in a diffusion region in the immobilized layer (thickness d) except an initial surface charging current region. In this equation, n = 2 (document value), A = 0.071 $cm^2$, and d = 53 $\mu$m.

$$\text{Cottrell equation: } i(t) = nFAC(D/\pi t)^{1/2} \qquad (1)$$

i: current, t: time, n: number of electrons, F: Faraday constant, A: electrode surface area, C: concentration, D: diffusion constant

The concentration of the electron mediator other than $VK_3$ can be determined by the same method as described above. A method of determining the concentration of the electron mediator other than the electrochemical method is a method including eluting the immobilized electron mediator into a solvent by a chemical or physical method and determining the concentration by liquid chromatography and gas chromatography.

Then, the method for measuring the Michaelis constant $K_m$ and rate constant $k_{cat}$ of the enzymatic reaction using the electron mediator as a substrate is described.

The following electrochemical equation is established between the Michaelis constant $K_m$ and rate constant $k_{cat}$ (R. Matsumoto et al, J. Electroanal. Chem. 535 (2002)37).

$$\frac{I_s^{lim}}{n_M FA \sqrt{(n_S / n_M) D_M k_{cat} K_m [E]}} = \sqrt{2 \left[ \frac{[M]^*}{K_m} - \ln\left(1 + \frac{[M]^*}{K_m}\right)\right]} \qquad (2)$$

wherein $I_s^{lim}$ is the current value of a flat portion of a curve obtained by cyclic voltammetry, $n_M$ is the number of electrons of the electron mediator, $n_S$ is the number of electrons of the substrate, F is the Faraday constant, A is the electrode surface area, $D_M$ is the diffusion constant of the substrate, [E] is the enzyme concentration, and $[M]^*$ is the concentration of the electron mediator.

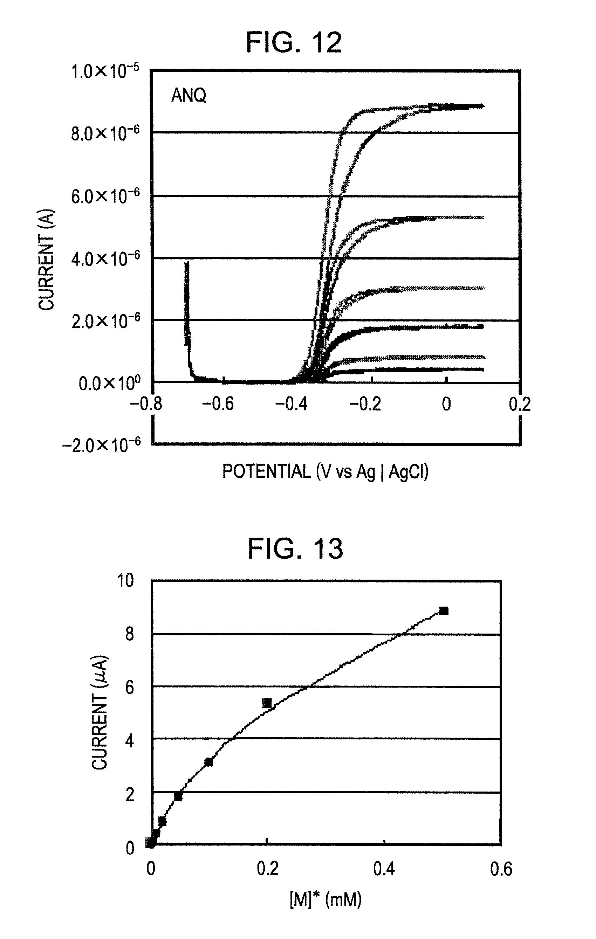

Fig. 12 shows the results of cyclic voltammetry performed by changing the concentration of ANQ used as the electron mediator in a solution containing sufficient amounts of NADH and DI. Fig. 13 shows plots of the current of the flat portion of the curve shown in Fig. 12 against the concentration of the electron mediator.

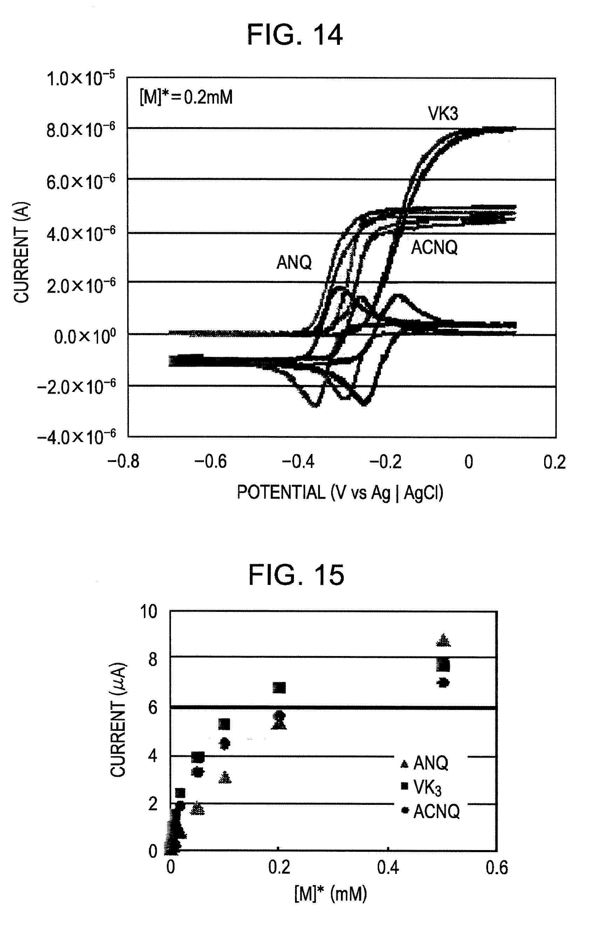

Fig. 14 shows the results of cyclic voltammetry performed at the constant concentrations $[M]^*$ of 0.2 mM of $VK_3$, ACNQ, and ANQ used as the electron mediator in a solution containing sufficient amounts of NADH and DI. Fig. 15 shows plots of the current of the flat portion of the curve shown in Fig. 14 against the concentration of the electron mediator.

Table 1 shows the results of $K_m$ and $K_{cat}$ determined from the equation (2) and Figs. 12 to 15. The value of ten times the thus-determined $K_m$ is 4 $\mu$M for $VK_3$, 20 $\mu$M for ACNQ, and 360 $\mu$M for ANQ.

| | $E^{O'}$ vs Ag \| AgCl | $k_{cat}$ ( $s^{-1}$ ) | $K_m$ ( $\mu$M ) | $k_{cat} / K_m$ |
|---|---|---|---|---|
| $VK_3$ | -0.22 | 630 | 0.4 | $1.6 \times 10^9$ |
| ACNQ | -0.26 | 400 | 2 | $2.0 \times 10^8$ |
| ANQ | -0.35 | 310 | 36 | $8.6 \times 10^6$ |

Next, the results of experiments conducted for evaluating the cathode 2 of the biofuel cell will be described.

Two CF sheets were laminated to prepare a CF electrode, the CF sheets being the same as those used for preparing the CF bio-anode, and BOD and $K_3[Fe(CN)_6]$ were immobilized on the CF electrode using PLL according to the following procedures.

On the CF electrode, 80 $\mu$l of an aqueous $K_3[Fe(CN)_6]$ solution (0.1 M), 80 $\mu$l of an aqueous PLL solution (1% (w/v)), and 80 $\mu$l of a BOD solution (50 mg/ml in a 50 mM phosphate buffer solution (pH 7.0)) were added in that order. After each of the addition steps, drying was performed at 30°C for 10 minutes. Before electrochemical measurement, the CF electrode (referred to as a "CF bio-cathode" hereinafter) having an immobilized layer formed thereon as described above was rinsed with distilled water for 5 minutes. As a result of measurement of the thickness of the CF bio-cathode by the same method as for the CF bio-anode before the electrochemical measurement, the thickness was 0.905 mm.

BOD (EC1. 3. 3. 5) derived from Myrothecium sp., which was used as BOD, was purchased from Amano Enzyme Inc. (Japan) and directly used without further purification. $K_3[Fe(CN)_6]$ was purchased from Wako Pure Chemical Industries, Ltd. (Japan). PLL (Mw = about 8,000) was purchased from Peptide Institute Inc., Ltd. (Japan).

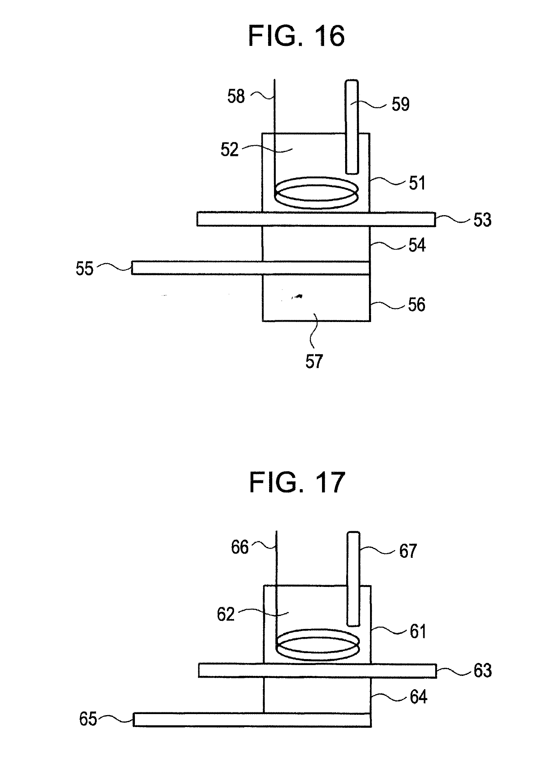

In order to evaluate the efficiency of material transport of oxygen ($O_2$) to the CF bio-cathode, two types of electrochemical measurement cells shown in Figs. 16 and 17 were prepared. In the electrochemical measurement cell shown in Fig. 16, a measurement solution (electrolyte solution) 52 placed in a vessel 51 is brought into contact with a cellophane film 53 at the bottom of the vessel 51. The CF bio-cathode as a working electrode 54 is brought into contact with the surface of the cellophane film 53 opposite to the measurement solution 52. A Ti mesh 55 is electrically connected to the working electrode 54. The CF bio-cathode as the working electrode 54 is immersed in a measurement solution (electrolyte solution) 57 placed in a vessel 56, and $O_2$ is supplied from outside air to the CD bio-cathode as the working electrode 54 through the measurement solution 57. The volumes of the measurement solutions 52 and 57 are 3 ml and 2 ml, respectively. Further, a Pt wire as a counter electrode 58, and an Ag|AgCl|saturated KCl electrode as a reference electrode 59 are immersed in the measurement solution 52. An electrochemical measurement device (not shown) was connected to the working electrode 54, the counter electrode 58, and the reference electrode 59. The electrochemical measurement cell is referred to as the "sink-type cell" in the sense that the CF bio-cathode as the working electrode 54 is immersed in the measurement solution 57. On the other hand, in the electrochemical measurement cell shown in Fig. 17, a measurement solution (electrolyte solution) 62 placed in a vessel 61 is brought into contact with a cellophane film 63 at the bottom of the vessel 61. The CF bio-cathode as a working electrode 64 is brought into contact with the surface of the cellophane film 63 opposite to the measurement solution 62. In this case, the cellophane film 63 has the function to control the supply of the measurement solution 62 to the CF bio-cathode. A Ti mesh 65 is electrically connected to the working electrode 64. The CF bio-cathode as the working electrode 64 is exposed to air. Therefore, $O_2$ can be diffused from air into the CF bio-cathode as the working electrode 64 through the Ti mesh 65. The volume of the measurement solution 62 is 3 ml. Further, a Pt wire as a counter electrode 66 and an Ag|AgCl|saturated KCl electrode as a reference electrode 67 are immersed in the measurement solution 62. An electrochemical measurement device (not shown) is connected to the working electrode 64, the counter electrode 66, and the reference electrode 67. The electrochemical measurement cell is referred to as the "open-air type cell" in the sense that the CF bio-cathode as the working electrode 64 is exposed to air.

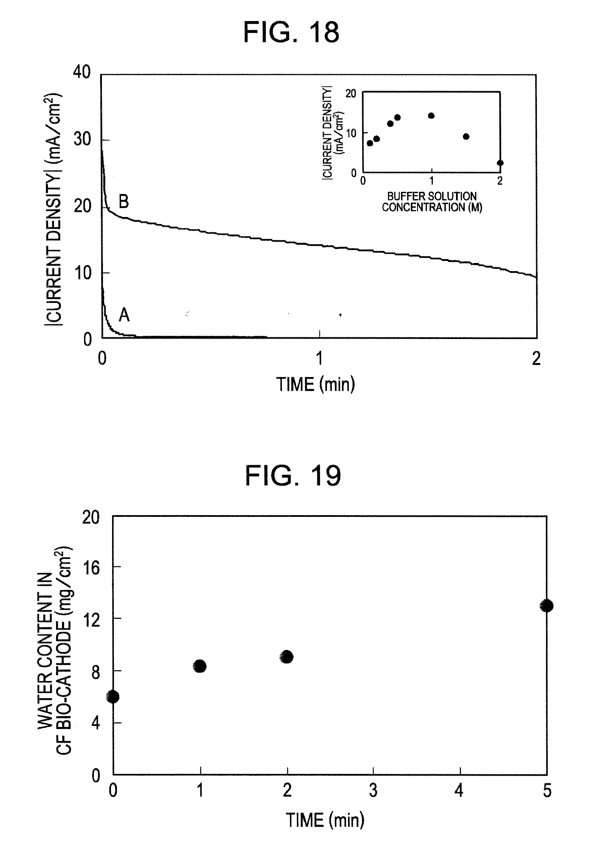

Fig. 18 shows the results (curve A) of chronoamperometry performed under a static condition using the sink-type cell shown in Fig. 16 and the results (curve B) of chronoamperometry performed under a static condition using the open-air-type cell shown in Fig. 17. A 0.1 M phosphate buffer solution (pH 7.0) containing of 0.4 M of glucose was used as each of the measurement solutions 52, 57, and 62. The measurement was performed at 0 V vs. Ag|AgCl. A graph inserted into Fig. 18 shows the results of measurement of the current density after chronoamperometry using the open-air type cell shown in Fig. 17 for 1 minute at 0 V with changing concentrations of the phosphate buffer solution (pH 7.0). As shown in Fig. 18, in the sink-type cell in which $O_2$ is supplied through the measurement solution 57, the current density on the FC bio-cathode rapidly deceases in chronoamperometry at 0 V in the 1.0 M phosphate buffer solution (pH 7.0) and comes close to zero after chronoamperometry for 1 minute (curve A). The significant decrease of the current density is due to insufficient supply of $O_2$. The concentration and diffusion constant of $O_2$ dissolved in an aqueous solution are 0.229 mM and 2.07 $\pm 0.66 \times 10^{-5}$ cm$^2$/s, respectively. On the other hand, in the open-air type cell, the current density after chronoamperometry for 1 minute is 14.1 mA/cm$^2$ (curve B in Fig. 18). This is due to the fact that $O_2$ is sufficiently supplied to the CF bio-cathode from air because the concentration (9 mM) in air and diffusion constant (the order of $10^{-1}$ cm$^2$/s) in gas are extremely higher than those in an aqueous solution. This indicates that the open-air type cell is effective for supplying $O_2$. In fact, the measurement of the water content in the CF bio-cathode of the open-air type cell produces the results as shown in Fig. 19. Namely, the water content after chronoamperometry for 1 minute is only 8.28 mg/cm$^2$. This indicates that in order to promote material transport of oxygen to the CF bio-cathode from air and cause an effective enzymatic reaction in the immobilized layer, it is important for the CF bio-cathode to be put into a partially wet condition. The water content was measured by a Karl Fisher method using a hygrometer (CA-200, Mitsubishi Chemical Co., Japan).

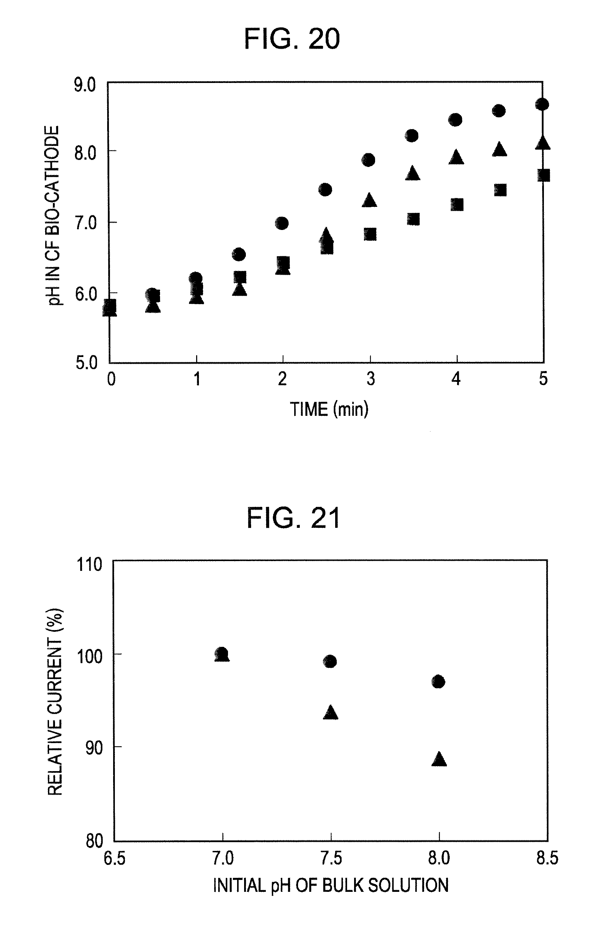

The current density on the CF bio-cathode also depends on the buffer solution concentration (the diagram inserted into Fig. 18). The profile of changes in current density with changes in the buffer solution concentration is very similar to that on the CF bio-anode shown in Fig. 7. In this case, the optimum value basically depends on the types of the enzyme, the electrode, the immobilized layer, and the electrolyte, but similarly, the optimum value is obtained when the buffer solution concentration is 1.0 M. This can be explained by a trade-off relation between insufficient proton diffusion and decrease in enzyme activity on the CF bio-anode as shown in Figs. 20, 21, and 22 which show the same results as Figs. 8, 9, and 10, respectively. In Fig. 20, • shows the buffer solution concentration of 0.1M, ▲ shows the buffer solution concentration of 1.0 M, and ■ shows the buffer solution concentration of 2.0 M. In Fig. 21, • shows the results after 1 minute and ▲ shows the results after 5 minutes.

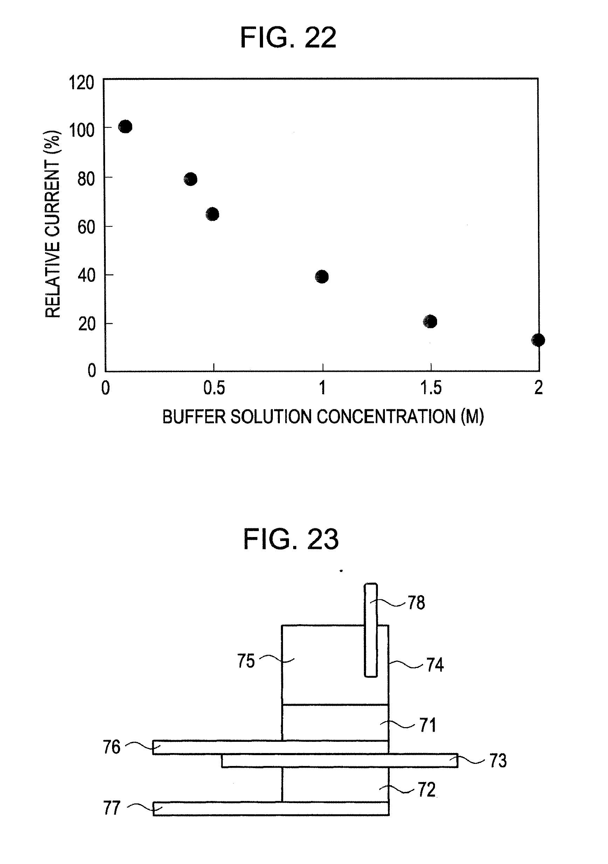

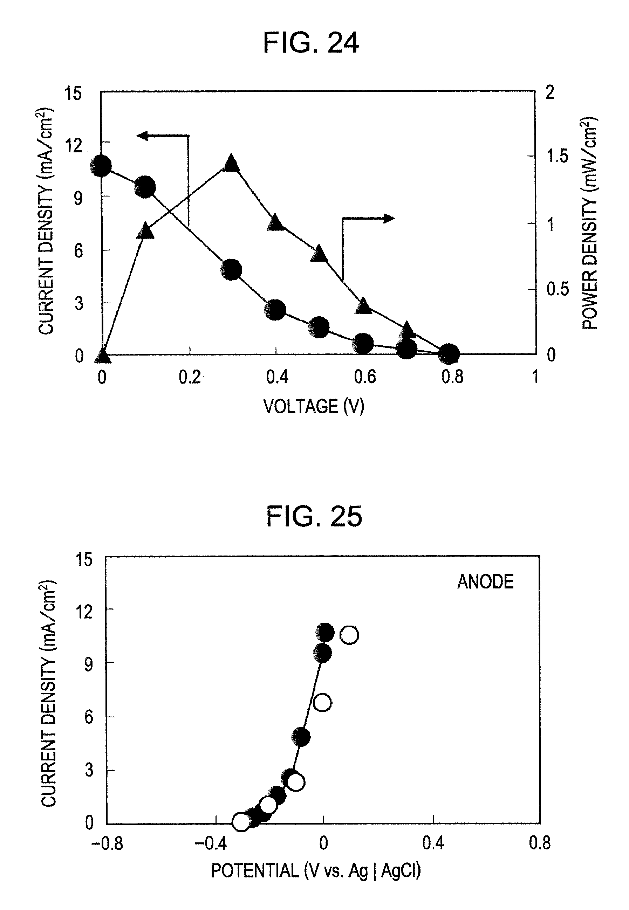

As shown in Fig. 23, a passive-type biofuel cell was prepared, in which a cellophane film 73 was sandwiched between a CF bio-anode 71 and a CF bio-cathode 72. A fuel solution (electrolyte solution) 75 was placed in a vessel 74. A Ti mesh was used as each of a current collector 76 for electric contact with the CF bio-anode 71 and a current collector 77 for electric contact with the CF bio-cathode 72. An Ag|AgCl|saturated KCl electrode was immersed as a reference electrode 78 in the fuel solution 75. The volume of the fuel solutions 75 was 3 ml. In order to evaluate the biofuel cell, a 1.0 M phosphate buffer solution (pH 7.0) containing 0.4 M of glucose was used as the fuel solution 75. Fig. 24 shows the output characteristics of the biofuel cell. The power density (▲ in Fig. 24) of the biofuel cell was determined from the current density (• in Fig. 24) after chronoamperometry of the biofuel cell for 1 minute at each voltage. The maximum power density was $1.45 \pm 0.24$ mW/cm$^2$ at 0.3 V, the open-circuit voltage was 0.8 V, and the short-circuit current density was 11 mA/cm$^2$.

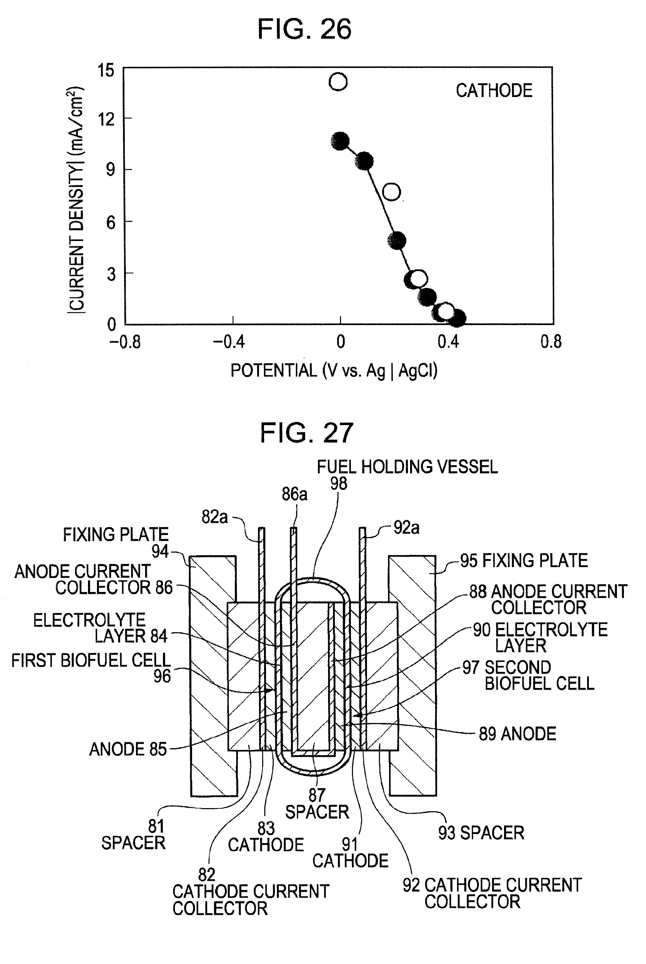

Figs. 25 and 26 show comparisons between the current density-potential curves (•) of the CF bio-anode 71 and the CF bio-cathode 72 during power generation of the biofuel cell shown in Fig. 23 and the current density-potential curves (○) of the CF bio-anode of the electrochemical measurement cell shown in Fig. 6 and the CF bio-cathode of the electrochemical measurement cell shown in Fig. 17, respectively. Figs. 25 and 26 indicate that the current densities on the CF bio-anode 71 and the CF bio-cathode 72 of the biofuel cell determined at each potential are substantially equivalent to those of the CF bio-anode of the electrochemical measurement cell shown in Fig. 6 and the CF bio-cathode of the electrochemical measurement cell shown in Fig. 13, respectively. This means that in the biofuel cell shown in Fig. 23, a voltage drop due to cell resistance is negligible even at a mA-order current density.

As described above, according to the first embodiment, the concentration of the electron mediator immobilized together with the enzyme on the electrode 11 of the anode 1 is at least 10 times the Michaelis constant $K_m$ of the electron mediator for the enzyme immobilized on the electrode 11, particularly for the coenzyme oxidase, the constant being determined by measurement in a solution. For example, when diaphorase is used as the coenzyme oxidase, the concentration of the electron mediator immobilized on the electrode 11 is selected to be 4 $\mu$M or more in use of VK$_3$ as the electron mediator, 20 $\mu$M or more in use of ACNQ as the electron mediator, and 360 $\mu$M or more in use of ANQ as the electron mediator. Therefore, the electron mediator can easily move in the immobilized layer 12 by diffusion while staying in the immobilized layer 12, thereby permitting rapid electron transfer reaction between diaphorase and the electrode 11 through the electron mediator. In addition, the enzyme activity in the immobilized layer 12 can be maintained, and thus the electrode reaction involving the enzyme, the coenzyme, and the electron mediator can be efficiently and stationarily effected. Consequently, the current density or power density of the biofuel cell can be improved. In addition, since the electrolyte layer 3 contains a sufficiently high concentration of buffer solution, the sufficient buffer ability can be achieved. Therefore, even when the amount of protons is increased or decreased in the immobilized layer 12 or the electrode 11 by enzymatic reaction through the protons in a high-output operation of the biofuel cell, the sufficient buffer function can be achieved, and a deviation of pH from proper pH can be sufficiently suppressed. As a result, the ability inherent to the enzyme can be sufficiently exhibited, and the electrode reaction involving the enzyme, the coenzyme, and the electron mediator can be efficiently and stationarily effected. Further, by using the CF bio-cathode as the cathode 2, oxygen can be efficiently supplied from outside air to the immobilized layer 22 on the electrode 21. Therefore, a current density over 10 mA/cm$^2$ can be obtained even under a resting condition. For example, a high-output passive-type biofuel cell capable of producing an output of 50 mW even with a cell volume of 40 can be realized. The biofuel cell is preferably applied to a power supply of a mobile electronic apparatus such as a portable music player or the like.

Next, a stacked biofuel cell unit according to a second embodiment of the present invention will be described.

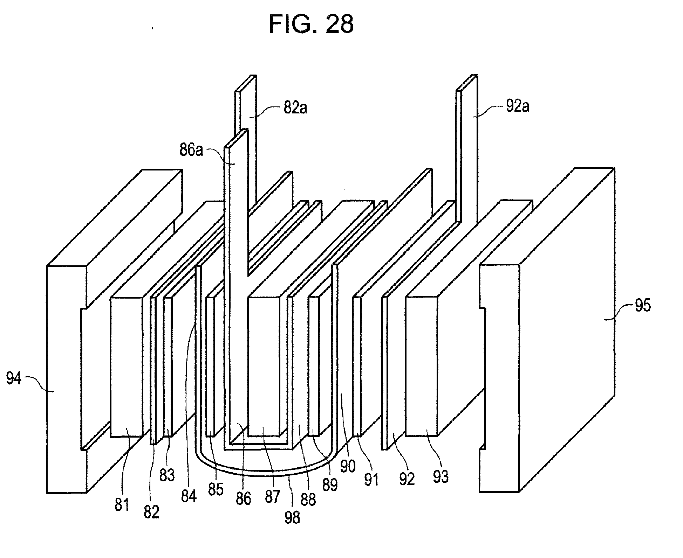

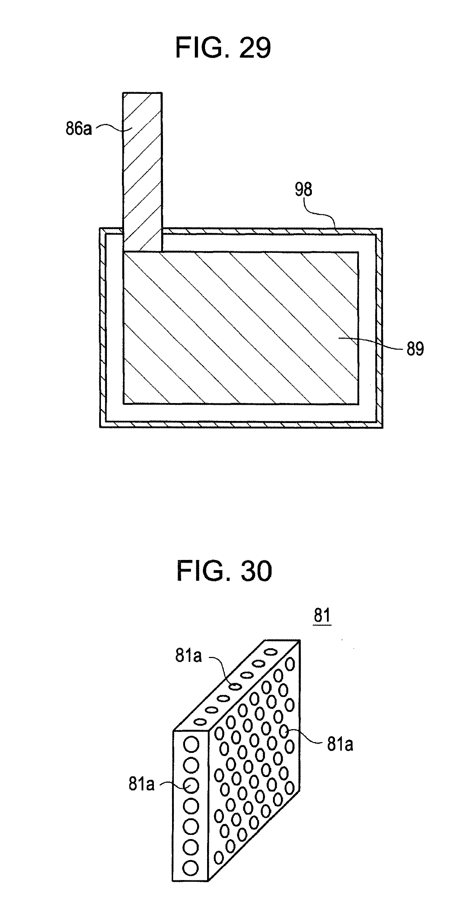

As shown in Figs. 27 and 28, in the stacked biofuel cell unit, a spacer 81, a cathode current collector 82, a cathode 83, an electrolyte layer 84, an anode 85, an anode current collector 86, a spacer 87, an anode current collector 88, an anode 89, an electrolyte layer 90, a cathode 91, a cathode current collector 92, and a spacer 93 are stacked in order, and the outer surfaces of the spacers 81 and 93 are held between fixing plates 94 and 95, the fixing plates 94 and 95 being fastened together with a screw (not shown) to fix these components. A first biofuel cell 96 is configured by a structure in which the cathode 83 and the anode 85 are opposed to each other with the electrolyte layer 84 provided therebetween. A second biofuel cell 97 is configured by a structure in which the cathode 91 and the anode 89 are opposed to each other with the electrolyte layer 90 provided therebetween. The spacer 81, the cathode 83, the anode 85, the spacer 87, the anode 89, the cathode 91, and the spacer 93 have substantially the same shape and size and are formed in, for example, a rectangular plate shape. However, the shape is not limited to this. In this case, the anode current collector 86 and the anode current collector 88 are integrally formed but the shape is not limited to this. The cathode current collector 82, the anode current collector 86, and the cathode current collector 92 have elongated terminals 82a, 86a, and 92a, respectively. Reference numeral 98 denotes a fuel holding vessel. The fuel to be used, for example, a glucose solution, is placed in the fuel holding vessel 98. The fuel holding vessel 98 is integrated with the electrolyte layers 84 and 90 to have a closed bag-like shape as a whole. The bag-like fuel holding vessel 98 integrated with the electrolyte layers 84 and 90 wraps the anode 85, the anode current collector 86, the spacer 87, the anode current collector 88, and the anode 89 so that the fuel is held in contact with the anodes 85 and 89. The terminal 86a of the anode current collector 86 extends outward from the fuel holding vessel 98. The portion between the terminal 86a and the fuel holding vessel 98 is sealed with, for example, a sealing member (not shown) so as to prevent leakage of the fuel to the outside from the portion. Fig. 29 shows an example of the shape of the fuel holding vessel 98 as viewed in the stacking direction.

The spacers 81 and 83 have the function to take in a gas containing at least an oxidizer, for example, a gas such as air, oxygen, or the like, from the outside and the function to diffuse the gas. Specifically, the spacers 81 and 93 have a structure permeable to the gas and are composed of, for example, an electrically insulating material, e.g., a plastic. Each of the spacers 81 and 93 preferably has a gap communicating throughout the entirety so that the gas supplied to any one of the surfaces can be discharged from another surface. However, the structure is not limited to this as long as the gas supplied to any surfaces other than the surfaces of the spacers 81 and 93 which face at least the cathodes 82 and 91, respectively, is discharged from the surfaces facing the cathodes 82 and 91, respectively. More specifically, the spacers 81 and 93 have many through holes which are regularly or irregularly arranged in each of the surfaces, and the through holes may have the same shape or different shapes. Fig. 30 shows an example in which many through holes 81a are regularly formed in the spacer 81. The spacer 93 has the same shape. The spacers 81 and 93 can be easily formed using, for example, an acrylic plate.

The spacer 87 has the function to hold the fuel, for example, the glucose solution, i.e., the function as a fuel tank, and the function to diffuse the fuel. Specifically, the spacer 87 has a structure permeable to the fuel and is composed of, for example, an electrically insulating material, e.g., a plastic. The spacer 87 preferably has a gap communicating throughout the entirety so that the fuel supplied to any one of the surfaces can be discharged from another surface. However, the structure is not limited to this as long as the fuel in contact with any one surface other than the surfaces of the spacer 87 which face at least the anodes 85 and 89 is discharged from the surfaces facing the anodes 85 and 89. More specifically, the spacer 87 has many through holes which are regularly or irregularly arranged in each of the surfaces, and the through holes may have the same shape or different shapes. An example of the spacer 87 is the same as shown in Fig. 30. Like the spacers 81 and 93, the spacer 87 can be easily formed using, for example, an acrylic plate.

The cathode current collectors 82 and 92 are adapted to collect currents generated in the cathodes 83 and 91, respectively, and the currents are taken out to the outside through the terminals 82a and 92a, respectively. In order that a gas containing an oxidizer can be supplied to the cathodes 83 and 91 through the cathode current collectors 82 and 92, respectively, the cathode current collectors 82 and 92 are composed of a conductor having a structure permeable to the gas, specifically, a mesh made of a metal, for example, titanium. Similarly, the anode current collectors 86 and 88 are adapted to collect currents generated in the anodes 85 and 89, respectively, and the currents are taken out to the outside through the terminal 86a. In order that the fuel can be supplied to the anodes 85 and 89 through the anode current collectors 86 and 88, respectively, the anode current collectors 86 and 88 are composed of a conductor having a structure permeable to the fuel, specifically, a mesh made of a metal, for example, titanium, like the cathode current collectors 82 and 92.

The anodes 85 and 89 have the same configuration as the anode 1 of the first embodiment, and the cathodes 83 and 91 have the same configuration as the cathode 2 of the first embodiment.

The fixing plates 94 and 95 are composed of a high-elasticity material, for example, a metal such as hard aluminum, so that the fixing plates 94 and 95 can be fastened together with, for example, a screw to securely fix together the components of the fuel cell. However, the configuration is not limited to this. The fastening positions of the fixing plates 94 and 95 are preferably selected so that a force can be uniformly applied to the components of the fuel cell.

In the stacked biofuel cell unit configured as described above, for example, when a glucose solution is used as the fuel placed in the fuel holding vessel 98, on the anodes 85 and 89, the glucose is degraded with the enzyme to take out electrons and produce $H^+$. On the cathodes 83 and 91, water is produced from H+ transported from the anodes 85 and 99 through the electrolyte layers 84 and 90, respectively, electrons transferred form the anodes 85 and 89 through an external circuit, and oxygen, for example, in air. As result, an output voltage can be obtained between the terminals 82a and 86a of the first biofuel cell 86 having the structure in which the cathode 83 and the anode 85 are opposed to each other with the electrolyte layer 84 provided therebetween. At the same time, an output voltage can be obtained between the terminals 92a and 86a of the second biofuel cell 97 having the structure in which the cathode 91 and the anode 89 are opposed to each other with the electrolyte layer 90 provided therebetween. Therefore, for example, when loads are connected between the terminal 86a and the terminals 82a and 92a of the stacked biofuel cell unit, a total of the output currents of the first and second biofuel cells 96 and 97 can be caused to flow to the loads.

As described above, according to the second embodiment, the stacked biofuel cell unit is configured so that the spacer 81 permeable to the gas containing the oxidizer, the cathode current collector 82, the cathode 83, the electrolyte layer 84, the anode 85, the anode current collector 86, the spacer 87 permeable to the fuel, the anode current collector 88, the anode 89, the electrolyte layer 90, the cathode 91, the cathode current collector 92, and the spacer 93 permeable to the gas containing the oxidizer are stacked in order, and the fuel holding vessel 98 is provided to wrap the anode 85, the anode current collector 86, the spacer 87, the anode current collector 88, and the anode 89. Therefore, the first biofuel cell 96 and the second biofuel cell 97 can be integrated in the most compact form under a condition in which the gas containing the oxidizer can be supplied to the cathodes 83 and 91, and the fuel can be supplied to the anodes 85 and 89. In addition, according to the stacked biofuel cell unit, higher output current and power can be obtained as compared with conventional fuel cell units. For example, with a cell volume of 80 $cm^3$ or more, an output of 100 mW or more can be obtained, and the output can be significantly increased. In fact, operations of a portable music player and a radio-controlled car were succeeded using the stacked biofuel cell unit as a power supply.

Although the embodiments of the present invention are described in detail above, the present invention is not limited to these embodiments, and various modifications can be made on the basis of the technical idea of the present invention.

For example, the numerical values, structures, configurations, shapes, and materials which are described in the embodiments are only examples, and different numerical values, structures, configurations, shapes, and materials may be used according to demand.

## Claims

1. A biofuel cell having a structure in which a cathode and an anode are opposed to each other with a proton conductor provided therebetween, at least one of the cathode and the anode including an electrode on which at least one enzyme and at least one electron mediator are immobilized,
wherein the concentration of the electron mediator immobilized on the electrode is at least 10 times a Michaelis constant $K_m$ of the electron mediator for the enzyme, which is determined by measurement in a solution.

2. The biofuel cell according to Claim 1, wherein the enzyme is diaphorase, the electron mediator is a quinone-type electron mediator, and a method for immobilizing the enzyme and the electron mediator on the electrode is a polyion complex method.

3. The biofuel cell according to Claim 2, wherein the electron mediator is $VK_3$, and the concentration of the electron mediator immobilized on the electrode is 4 $\mu$M or more.

4. The biofuel cell according to Claim 2, wherein the electron mediator is ACNQ, and the concentration of the electron mediator immobilized on the electrode is 20 $\mu$M or more.

5. The biofuel cell according to Claim 2, wherein the electron mediator is ANQ, and the concentration of the electron mediator immobilized on the electrode is 360 $\mu$M or more.

6. The Biofuel cell according to Claim 2, wherein the electron mediator is $VK_3$, and the concentration of the electron mediator immobilized on the electrode is 28 $\mu$M or more.

7. The biofuel cell according to Claim 2, wherein the enzyme immobilized on the electrode of the anode contains an oxidase which promotes oxidation of a monosaccharide and degrades it.

8. The biofuel cell according to Claim 7, wherein the enzyme immobilized on the electrode of the anode contains a coenzyme oxidase which returns a coenzyme reduced with oxidation of the monosaccharide to an oxidized form and which transfers electrons to the anode through the electron mediator.

9. The biofuel cell according to Claim 7, wherein the monosaccharide is glucose, and the oxidase is glucose dehydrogenase.

10. A method for producing a biofuel cell having a structure in which a cathode and an anode are opposed to each other with a proton conductor provided therebetween, at least one of the cathode and the anode including an electrode on which at least one enzyme and at least one electron mediator are immobilized, the method comprising controlling the concentration of the electron mediator immobilized on the electrode to be at least 10 times a Michaelis constant $K_m$ of the electron mediator for the enzyme, which is determined by measurement in a solution.

11. An electronic apparatus comprising at least one biofuel cell,
wherein the biofuel cell has a structure in which a cathode and an anode are opposed to each other with a proton conductor provided therebetween,

at least one of the cathode and the anode includes an electrode on which at least one enzyme and at least one electron mediator are immobilized, and

the concentration of the electron mediator immobilized on the electrode is at least 10 times a Michaelis constant $K_m$ of the electron mediator for the enzyme, which is determined by measurement in a solution.

**12.** An enzyme-immobilized electrode comprising an electrode on which at least one enzyme and at least one electron mediator are immobilized,

wherein the concentration of the electron mediator immobilized on the electrode is at least 10 times a Michaelis constant $K_m$ of the electron mediator for the enzyme, which is determined by measurement in a solution.

**13.** A method for producing an enzyme-immobilized electrode comprising producing an electrode on which at least one enzyme and at least one electron mediator are immobilized,

wherein the concentration of the electron mediator immobilized on the electrode is at least 10 times a Michaelis constant $K_m$ of the electron mediator for the enzyme, which is determined by measurement in a solution.

# FIG. 1

GLUCOSE    GLUCONOLACTONE

− ———

1 ANODE

3 ELECTROLYTE LAYER

+ ———

2 CATHODE

O$_2$    H$_2$O

# FIG. 2

GLUCOSE → GLUCONOLACTONE

GLUCOSE DEHYDROGENASE (GDH)

NADH → NAD$^+$

DIAPHORASE (DI)

VK$_3$(ox) → VK$_3$(red)

ELECTRON FLOW

1 ANODE

12

11 ELECTRODE

3 ELECTROLYTE LAYER

21 ELECTRODE

Fe(CN)$_6^{4-}$ → Fe(CN)$_6^{3-}$

BILIRUBIN OXIDASE (BOD)

O$_2$ → H$_2$O

2 CATHODE

22

## FIG. 3

# FIG. 4

# FIG. 5

# FIG. 6

# FIG. 7

## FIG. 8

## FIG. 9

# FIG. 10

# FIG. 11

## FIG. 12

## FIG. 13

## FIG. 14

## FIG. 15

FIG. 16

FIG. 17

# FIG. 18

# FIG. 19

## FIG. 20

## FIG. 21

# FIG. 22

# FIG. 23

# FIG. 24

# FIG. 25

# FIG. 26

# FIG. 27

FUEL HOLDING VESSEL 98

FIXING PLATE 94
ANODE CURRENT COLLECTOR 86
ELECTROLYTE LAYER 84
FIRST BIOFUEL CELL 96
ANODE 85
81 SPACER
82 CATHODE CURRENT COLLECTOR
83 CATHODE
87 SPACER
91 CATHODE

82a
86a

92a
95 FIXING PLATE
88 ANODE CURRENT COLLECTOR
90 ELECTROLYTE LAYER
97 SECOND BIOFUEL CELL
89 ANODE
93 SPACER
92 CATHODE CURRENT COLLECTOR

FIG. 28

# FIG. 29

86a

98

89

# FIG. 30

81

81a

81a

81a

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2004071559 A
- JP 2005013210 A
- JP 2005310613 A
- JP 2006024555 A
- JP 2006049215 A
- JP 2006093090 A
- JP 2006127957 A
- JP 2006156354 A
- JP 2007012281 A
- JP 2007035437 A
- JP 2007087627 A
- JP 2007188810 A

### Non-patent literature cited in the description

- **HELLER, A.** Miniature biofuel cells. *Phys. Chem. Chem. Phys.,* 2004, vol. 6, 209-216
- **BARTON, S. C. ; GALLAWAY, J. ; ATANASSOV, P.** Enzymatic biofuel cells for implantable and micro-scale devices. *Chem. Rev.,* 2004, vol. 104, 4867-4886
- **PALMORE, G. T. R. ; WHITESIDES, G. M.** Microbial and enzymatic biofuel cells. *ACS Symp. Ser.,* 1994, vol. 556, 271-290
- **KANO, K. ; IKEDA, T.** Bioelectrocatalysis, Powerful means of connecting electrochemistry to biochemistry and biotechnology. *Electrochemistry,* 2003, vol. 71 (2), 86-99
- **KATZ, E. ; WILLNER, I.** A biofuel cell with electrochemically switchable and tunable power output. *J. Am. Chem. Soc.,* 2003, vol. 125, 6803-6813
- **KAMITAKA, Y. ; TSUJIMURA, S. ; SETOYAMA, N. ; KAJINO, T. ; KANO, K.** Fructose/dioxygen biofuel cell based on direct electron transfer-type bioelectrocatalysis. *Phys. Chem. Chem. Phys.,* 2007, vol. 9 (15), 1793-1801
- **PALMORE, G. T. R. ; BERTSCHY, H. ; BERGENS, S. H. ; WHITESIDES, G. M.** A methanol/dioxygen biofuel cell that uses NAD(+)-dependent dehydrogenases as catalysts: Application of an electro-enzymatic method to regenerate nicotinamide adenine dinucleotide at low overpotentials. *J. Electroanal. Chem.,* 1998, vol. 443, 155-161
- **TOPCAGIC, S. ; MINTEER, S. D.** Development of a membraneless ethanol/oxygen biofuel cell. *Electrochimica Acta,* 2007, vol. 51, 2168-2172
- **TSUJIMURA, S. ; FUJITA, M. ; TATSUMI, H. ; KANO, K. ; IKEDA, T.** Bioelectrocatalysis-based dihydrogen/dioxygen fuel cell operating at physiological pH. *Phys. Chem. Chem. Phys.,* 2001, vol. 3, 1331-1335
- **MANO, N. ; MAO, F. ; HELLER, A.** Characteristics of a miniature compartment-less grucose-O2 biofuel cell and its operation in a living plant. *J. Am. Chem. Soc,* 2003, vol. 125, 6588-6594
- **TOGO, M. ; TAKAMURA, A. ; ASAI, T. ; KAJI, H. ; NISHIZAWA, M.** An enzyme-based microfluidic biofuel cell using vitamin K3-mediated glucose oxidation. *Electrochimica Acta,* 2007, vol. 52, 4669-4674
- **CHAUDHURI, S. K. ; LOVLEY, D. R.** Electricity generation by direct oxidation of glucose in mediatorless microbial fuel cells. *Nature Biotech,* 2003, vol. 21 (10), 1229-1232
- **LOGAN, B. E. ; AELTERMAN, P. ; HAMELERS, B. ; ROZENDAL, R. ; SCHROEDER, U. ; KELLER, J. ; FREGUIA, S. ; VERSTRAETE, W. ; RABAEY, K.** Microbial fuel cells: Methodology and technology. *Environmental Science & Technology,* 2006, vol. 9 (3), 5181-5192
- **BULLEN, R. A. ; ARNOT, T. C. ; LAKEMAN, J. B. ; WALSH, F. C.** Biofuel cells and their development. *Biosens. Bioelectron,* 2006, vol. 21, 2015-2045
- **SATO, A. ; KANO, K. ; IKEDA, T.** Diaphorase/naphtoquinone derivative-modified electrode as an anode for diffusion-controlled oxidation of NADH in electrochemical cells. *Chem. Lett.,* 2003, vol. 32, 880-881
- **R. MATSUMOTO et al.** *J. Electroanal. Chem.,* 2002, vol. 535, 37